(19) Europäisches Patentamt
European Patent Office
Office européen des brevets

(11) **EP 3 884 981 B1**

(12) **EUROPEAN PATENT SPECIFICATION**

(45) Date of publication and mention of the grant of the patent:
**10.07.2024 Bulletin 2024/28**

(21) Application number: **18940745.5**

(22) Date of filing: **23.11.2018**

(51) International Patent Classification (IPC):
***A61M 16/00*** (2006.01)

(52) Cooperative Patent Classification (CPC):
**A61M 16/024; A61M 16/0051;** A61M 16/021;
A61M 2202/0208; A61M 2230/00; A61M 2230/46

(86) International application number:
**PCT/CN2018/117323**

(87) International publication number:
**WO 2020/103163 (28.05.2020 Gazette 2020/22)**

(54) **POSITIVE END EXPIRATORY PRESSURE DETERMINING APPARATUS**

EINRICHTUNG ZUR BESTIMMUNG VON POSITIVEM ENDAUSATMUNGSDRUCK

APPAREIL DE DÉTERMINATION DE PRESSION POSITIVE EN FIN D'EXPIRATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**29.09.2021 Bulletin 2021/39**

(73) Proprietors:
• **Shenzhen Mindray Bio-Medical Electronics Co., Ltd.**
**Shenzhen, Guangdong 518057 (CN)**
• **Zhongda Hospital Southeast University**
**Nanjing, Jiangsu 210009 (CN)**

(72) Inventors:
• **LIU, Ling**
**Nanjing, Jiangsu 518057 (CN)**
• **LIU, Jinglei**
**Shenzhen, Guangdong 518057 (CN)**
• **YANG, Yi**
**Nanjing, Jiangsu 518057 (CN)**
• **ZHOU, Xiaoyong**
**Shenzhen, Guangdong 518057 (CN)**
• **QIU, Haibo**
**Nanjing, Jiangsu 518057 (CN)**
• **WAN, Congying**
**Shenzhen, Guangdong 518057 (CN)**

• **PAN, Chun**
**Nanjing, Jiangsu 518057 (CN)**
• **CHEN, Jun**
**Shenzhen, Guangdong 518057 (CN)**
• **XIE, Jianfeng**
**Nanjing, Jiangsu 518057 (CN)**

(74) Representative: **KIPA AB**
**Drottninggatan 11**
**252 21 Helsingborg (SE)**

(56) References cited:
EP-A1- 1 579 882      EP-A1- 2 246 087
WO-A1-2018/153964    WO-A1-2018/153964
CN-A- 102 369 036    CN-A- 103 608 062
CN-A- 106 462 660    CN-A- 107 970 510
US-A- 5 915 381      US-A1- 2005 109 340
US-A1- 2008 236 582  US-A1- 2012 055 476

• CHEN, WEIMING; LU, GUOPING: "Lung Recruitment Maneuvers and Positive End-Expiratory Pressure Titration in Children with Acute Respiratory Distress Syndrome", CHINESE JOURNAL OF CONTEMPORARY PEDIATRICS, vol. 20, no. 9, 30 September 2018 (2018-09-30), pages 706 - 712, XP055709763, ISSN: 1008-8830

**Description**

TECHNICAL FIELD

The disclosure relates to the technical field of medical equipments, and in particular to a method and an apparatus for determining positive end expiratory pressure, a ventilation device, and a storage medium.

BACKGROUND

Positive end expiratory pressure (PEEP) is one of the key parameters in ventilator therapy. The PEEP can increase the end-expiratory lung volume such that the alveoli are not prone to collapse at the end of expiration, and the PEEP plays an important role in the treatment of acute respiratory distress syndrome (ARDS), non-cardiogenic pulmonary oedema and pulmonary haemorrhage. The preferred PEEP required by each patient is different due to the different clinical condition of each patient. Excessively high or low PEEP may cause damage to and function indication of the brain, liver, kidney and other organs of a patient, so how to determine the preferred PEEP is a critical point and difficulty in clinical practice.

EP2246087A1 relates to system and methods for ventilating a patient, where the PEEP is used as a variable parameter during the ventilation process. In order to realize the pre-set tidal volume, when the peek airway pressure is fixed, the PEEP is automatically adjusted.

WO2018/153964 relates to automatic PEEP selection for mechanical ventilation. The system is configured to perform recruitment and/or continuous monitoring and adjustments of the PEEP setting to maintain an open lung, and the optimal PEEP is selected based the change of the lung compliance or the inflection point of a plotted curve between the lung volume and the transpulmonary pressure.

EP1579882A1 relates to non-invasive method for optimizing the respiration for atelectatic lungs. The method measures the $CO_2$ concentration, and traces values the airway pressure level at which alveolar opening or lung overdistension or lung open condition or alveolar closing occurs.

US20120055476 relates to apparatus for identifying FRC and PEEP characteristics. The amount of the lung volume recruited/de-recruited at various levels of PEEP may be determined for use in selecting a desired PEEP.

US20050109340 relates to method for controlling a ventilator. During the ventilation process, the $S_{pO2}$ is evaluated, and when the detected $S_{pO2}$ does not meet the predefined $S_{pO2}$, the PEEP is automatically regulated.

SUMMARY

In order to solve the technical problem, embodiments of the disclosure aim to provide an apparatus for determining positive end expiratory pressure, and a ventilation device, which can automatically determine a preferred PEEP. The invention is defined by the appended claims.

The technical solutions of the embodiments of the disclosure may be implemented as follows.

An example, not part of the invention, provides a positive end expiratory pressure determining method applicable to a ventilation device. The method comprises:

controlling a ventilation control module for stepwise changing a PEEP value for ventilation of the ventilation device to a patient;
controlling a physiological parameter monitoring module for monitoring at least one physiological parameter of the patient;
controlling a calculation module for calculating a change in the at least one physiological parameter every time the PEEP value is changed; and
determining a preferred PEEP value for ventilation to the patient on the basis of the change in the at least one physiological parameter;
the at least one physiological parameter comprises at least two of a driving pressure, a compliance, a dead space ratio and an oxygenation index, and determining a preferred PEEP value for ventilation to the patient according to the change in the at least one physiological parameter comprises: determining respective candidate PEEP values according to the changes in the respective at least two physiological parameters; and determining the preferred PEEP value for ventilation to the patient according to the respective candidate PEEP values;
the candidate PEEP values comprise any one of the PEEP value corresponding to a minimum value of the driving pressure and the PEEP value corresponding to a maximum value of the compliance, and at least one of the PEEP value corresponding to a minimum value of the dead space ratio and the PEEP value corresponding to a maximum point of a change rate of the oxygenation index;
where determining the preferred PEEP value for ventilation to the patient according to the respective candidate PEEP values comprises:

determining the preferred PEEP value according to the PEEP value corresponding to the minimum value of the dead space ratio and any one of the PEEP value corresponding to the minimum value of the driving pressure and the PEEP value corresponding to the maximum value of the compliance; or, determining the preferred PEEP value according to the PEEP value corresponding to the maximum point of the change rate of the oxygenation index and any one of the PEEP value corresponding to the minimum value of the driving pressure and the PEEP value corresponding to the maximum value of the compliance; or, determining the preferred PEEP value according to the PEEP value corresponding to the minimum value of the dead space ratio, the PEEP value corresponding to the maximum point of the change rate of the oxygenation index and any one of the PEEP value corresponding to the minimum value of the driving pressure and the PEEP value corresponding to the maximum value of the compliance..

In the above method, the at least one physiological parameter is a driving pressure; and
the step of determining a preferred PEEP value for ventilation to the patient on the basis of the change in the at least one physiological parameter comprises:
the PEEP value corresponding to a minimum value of the driving pressure being the preferred PEEP value as the PEEP value changes stepwise.

In the above method, the at least one physiological parameter is a compliance; and
the step of determining a preferred PEEP value for ventilation to the patient on the basis of the change in the at least one physiological parameter comprises:

the PEEP value corresponding to a maximum value of the compliance being the preferred PEEP value as the PEEP value changes stepwise.

In the above method, the at least one physiological parameter is a dead space ratio; and
the step of determining a preferred PEEP value for ventilation to the patient on the basis of the change in the at least one physiological parameter comprises:

the PEEP value corresponding to a minimum value of the dead space ratio being the preferred PEEP value as the PEEP value changes stepwise.

In the above method, the at least one physiological parameter is an oxygenation index; and
the step of determining a preferred PEEP value for ventilation to the patient on the basis of the change in the at least one physiological parameter comprises:

the PEEP value corresponding to a maximum point of a rate of change of the oxygenation index being the preferred PEEP value as the PEEP value changes stepwise.

In the above method, the at least one physiological parameter comprises: at least two of the driving pressure, the compliance, the dead space ratio and the oxygenation index, and the step of determining a preferred PEEP value for ventilation to the patient on the basis of the change in the at least one physiological parameter comprises:

determining respective candidate PEEP values on the basis of the changes in the respective physiological parameters; and
determining the preferred PEEP value for ventilation to the patient according to the respective determined candidate PEEP values.

In the above method, after the preferred PEEP value for ventilation to the patient is determined on the basis of the change in the at least one physiological parameter, the method further comprises:
outputting the preferred PEEP value according to a preset prompting strategy.

In the above method, after the preferred PEEP value for ventilation to the patient is determined on the basis of the change in the at least one physiological parameter, the method further comprises:
providing mechanical ventilation to the patient according to the preferred PEEP value.

An embodiment of the disclosure provides a positive end expiratory pressure determining apparatus applicable to a ventilation device. The apparatus comprises:

a ventilation control module for stepwise changing a PEEP value for ventilation of a ventilation device to a patient;
a physiological parameter monitoring module for monitoring at least one physiological parameter of the patient; and
a calculation module for calculating a change in the at least one physiological parameter every time the PEEP value changes and determining a preferred PEEP value for ventilation to the patient on the basis of the change in the at least one physiological parameter;
the at least one physiological parameter comprises at least two of a driving pressure, a compliance, a dead space ratio and an oxygenation index, and the calculation module is further configured for: determining respective candidate PEEP values according to the changes in the respective at least two physiological parameters; and determining the preferred PEEP value for ventilation to the patient according to the respective candidate PEEP values;

the candidate PEEP values comprise any one of the PEEP value corresponding to a minimum value of the driving pressure and the PEEP value corresponding to a maximum value of the compliance, and at least one of the PEEP value corresponding to a minimum value of the dead space ratio and the PEEP value corresponding to a maximum point of a change rate of the oxygenation index;

the calculation module is configured for determining the preferred PEEP value for ventilation to the patient according to the respective candidate PEEP values by:

determining the preferred PEEP value according to the PEEP value corresponding to the minimum value of the dead space ratio and any one of the PEEP value corresponding to the minimum value of the driving pressure and the PEEP value corresponding to the maximum value of the compliance; or, determining the preferred PEEP value according to the PEEP value corresponding to the maximum point of the change rate of the oxygenation index and any one of the PEEP value corresponding to the minimum value of the driving pressure and the PEEP value corresponding to the maximum value of the compliance; or, determining the preferred PEEP value according to the PEEP value corresponding to the minimum value of the dead space ratio, the PEEP value corresponding to the maximum point of the change rate of the oxygenation index and any one of the PEEP value corresponding to the minimum value of the driving pressure and the PEEP value corresponding to the maximum value of the compliance..

In the above apparatus, the at least one physiological parameter is a driving pressure, and the calculation module determines that the PEEP value corresponding to a minimum value of the driving pressure is the preferred PEEP value as the PEEP value changes stepwise.

In the above apparatus, the at least one physiological parameter is a compliance, and the calculation module determines that the PEEP value corresponding to a maximum value of the compliance is the preferred PEEP value as the PEEP value changes stepwise.

In the above apparatus, the at least one physiological parameter is a dead space ratio, and the calculation module determines that the PEEP value corresponding to a minimum value of the dead space ratio is the preferred PEEP value as the PEEP value changes stepwise.

In the above apparatus, the at least one physiological parameter is an oxygenation index, and the calculation module determines that the PEEP value corresponding to a maximum point of a rate of change of the oxygenation index is the preferred PEEP value as the PEEP value changes stepwise.

In the above apparatus, the at least one physiological parameter comprises: at least two of the driving pressure, the compliance, the dead space ratio and the oxygenation index, and the calculation module determines respective candidate PEEP values on the basis of the changes in the respective physiological parameters, and determines the preferred PEEP value for ventilation to the patient according to the respective determined candidate PEEP values.

In the above apparatus, the apparatus further comprises: an output module, wherein the output module outputs the preferred PEEP value according to a preset prompting strategy.

In the above apparatus, the apparatus further comprises: a ventilation control module, wherein the ventilation control module provides mechanical ventilation to the patient according to the preferred PEEP value.

An embodiment of the disclosure provides a ventilation device comprising any positive end expiratory pressure determining apparatus as described above, the ventilation device comprising a gas source, an inspiratory branch, an expiratory branch and a display, wherein

the gas source provides a gas during mechanical ventilation;

the inspiratory branch is connected to the gas source and provides an inspiratory path during the mechanical ventilation;

the expiratory branch provides an expiratory path during the mechanical ventilation;

the positive end expiratory pressure determining apparatus is connected to the inspiratory branch and the expiratory branch;

the positive end expiratory pressure determining apparatus determines a preferred PEEP value during the mechanical ventilation; and

the display is connected to the positive end expiratory pressure determining apparatus, and displays a respiratory waveform during the mechanical ventilation.

An embodiment of the disclosure provides a storage medium, wherein the storage medium stores a positive end expiratory pressure determining program, which is executable by a processor to implement any positive end expiratory pressure determining method as described above.

The embodiments of the disclosure provide a method and an apparatus for determining positive end expiratory pressure, a ventilation device, and a storage medium. The method may include: stepwise changing a PEEP value for ventilation of a ventilation device to a patient; monitoring at least one physiological parameter of the patient, calculating a change in the at least one physiological parameter every time the PEEP value changes, and determining a preferred PEEP value for ventilation to the patient according to the change in the at least one physiological parameter. By adopting the above solutions, the apparatus for determining positive end expiratory pressure provided by the embodiment of the disclosure stepwise changes the PEEP value, calculates the change in the at least one physiological parameter every time the PEEP value changes, and determines the preferred PEEP value for ventilation to the patient according to the change in the at least one physiological parameter. In this case, the apparatus for determining positive end expiratory pressure may determine different preferred PEEP values according to the physiological parameters of different patients to conduct mechanical ventilation, and can automatically determine the preferred PEEP values.

BRIEF DESCRIPTION OF THE DRAWINGS

FIG. 1 is a first flow chart of a method for determining positive end expiratory pressure provided according to an embodiment of the disclosure;
FIG. 2 is an exemplary profile of $P_{plat}$ as a PEEP value changes stepwise provided according to an embodiment of the disclosure;
FIG. 3 is an exemplary profile of a dead space ratio as a PEEP value changes stepwise provided according to an embodiment of the disclosure;
FIG. 4 is an exemplary profile of an oxygenation index as a PEEP value changes stepwise provided according to an embodiment of the disclosure;
FIG. 5 is a second flow chart of a method for determining positive end expiratory pressure provided according to an embodiment of the disclosure;
FIG. 6 is a third flow chart of a method for determining positive end expiratory pressure provided according to an embodiment of the disclosure;
FIG. 7 is a fourth flow chart of a method for determining positive end expiratory pressure provided according to an embodiment of the disclosure;
FIG. 8 is a fifth flow chart of a method for determining positive end expiratory pressure provided according to an embodiment of the disclosure;
FIG. 9 is a first schematic structural diagram of an apparatus for determining positive end expiratory pressure 1 provided according to an embodiment of the disclosure;
FIG. 10 is a second schematic structural diagram of an apparatus for determining positive end expiratory pressure 1 provided according to an embodiment of the disclosure;
FIG. 11 is a first schematic structural diagram of a ventilation device provided according to an embodiment of the disclosure; and
FIG. 12 is a second schematic structural diagram of a ventilation device provided according to an embodiment of the disclosure.

DESCRIPTION OF THE PREFERRED EMBODIMENTS

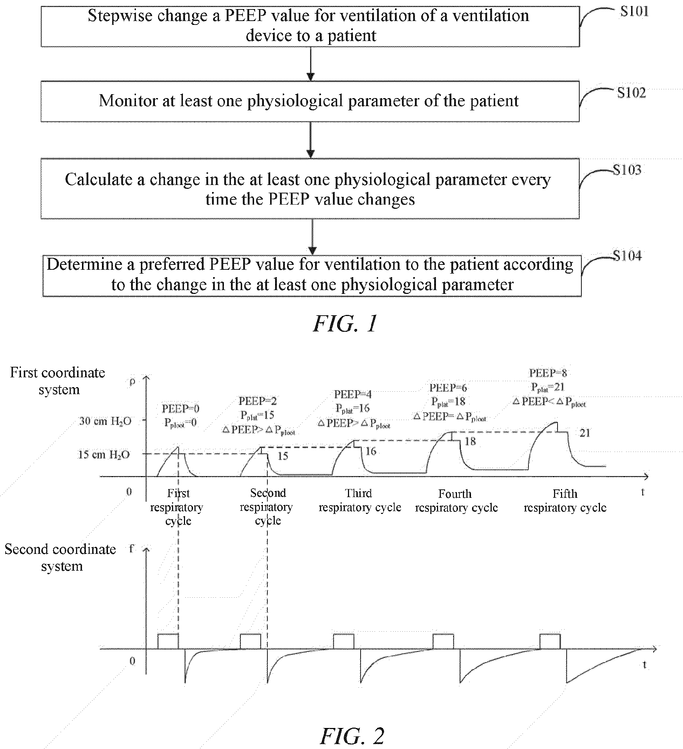

In order to understand the features and technical contents of embodiments of the disclosure in more detail, the implementation of the embodiments of the disclosure will be described below in detail with reference to the accompanying drawings, which are for reference and illustration only, and are not intended to limit the embodiments of the disclosure.
An embodiment of the disclosure provides a method for determining positive end expiratory pressure applicable to a ventilation device, where the ventilation device is a medical device having a ventilation function and may be a ventilator or an anaesthesia machine. The specific selection of the ventilation device is made according to the actual situation, which is not specifically limited in the embodiment of the disclosure. The method for determining positive end expiratory pressure provided according to the embodiment of the disclosure is applicable to the situation of mechanically ventilating a patient with a ventilation device. As shown in FIG. 1, the method may include the following steps.
At S101, a PEEP value for ventilation of a ventilation device to a patient is changed stepwise.
In the embodiment of the disclosure, the ventilation device can include an apparatus for determining positive end expiratory pressure, where the apparatus for determining positive end expiratory pressure may include a ventilation control module, a physiological parameter monitoring module, a calculation module and an output module. During the mechanical ventilation conducted on the patient by the ventilation device, the ventilation control module in the apparatus for determining positive end expiratory pressure may stepwise change the PEEP value for ventilation to the patient from an initial PEEP value sequentially in an ascending or descending order.

It should be noted that the initial PEEP value may be manually set by a doctor every time a PEEP titration is performed, or may be a preset value in the apparatus for determining positive end expiratory pressure, which is specifically selected according to the actual situation, and is not specifically limited in the embodiment of the disclosure.

At S 102, at least one physiological parameter of the patient is monitored.

After the ventilation control module of the apparatus for determining positive end expiratory pressure stepwise changes the PEEP value for ventilation of the ventilation device to the patient, the physiological parameter monitoring module in the apparatus for determining positive end expiratory pressure detects the at least one physiological parameter of the patient.

In the embodiment of the disclosure, the physiological parameter may include a driving pressure, a compliance, a dead space ratio, an oxygenation index, etc., and is specifically selected according to the actual situation, which is not specifically limited in the embodiment of the disclosure.

In the embodiment of the disclosure, during the PEEP titration, the doctor may select at least one physiological parameter from the driving pressure, the compliance, the dead space ratio and the oxygenation index, allowing the physiological parameter monitoring module in the apparatus for determining positive end expiratory pressure to monitor values of the at least one physiological parameter corresponding to different PEEP values.

The particular way in which the physiological parameter monitoring module in the apparatus for determining positive end expiratory pressure monitors the at least one physiological parameter of the patient is described in detail below.

In the embodiment of the disclosure, the physiological parameter monitoring module in the apparatus for determining positive end expiratory pressure may measure an airway plateau pressure value corresponding to the PEEP value; and a driving pressure value is calculated according to the PEEP value and the airway plateau pressure value. Specifically, the calculation of the driving pressure value according to the PEEP value and the airway plateau pressure value is as shown in Equation (1),

$$\Delta P = Pplat - PEEP \qquad (1)$$

where $\Delta P$ represents the driving pressure value, and Pplat represents the airway plateau pressure value.

In the embodiment of the disclosure, the physiological parameter monitoring module in the apparatus for determining positive end expiratory pressure may calculate a tidal volume according to an inspiratory duration and an inspiratory flow rate; and a compliance value is calculated according to the tidal volume, the PEEP value and the airway plateau pressure value. Here, the calculation of the compliance value is as shown in Equation (2),

$$Compliance = Tidal\ volume\ /\ (Pplat - PEEP) \qquad (2)$$

In the embodiment of the disclosure, the physiological parameter monitoring module in the apparatus for determining positive end expiratory pressure may measure a partial pressure value of carbon dioxide within the arterial blood and a partial pressure value of carbon dioxide in the expired gas corresponding to the PEEP value; and a dead space ratio value corresponding to the PEEP value is calculated according to the partial pressure value of carbon dioxide within the arterial blood and the partial pressure value of carbon dioxide in the expired air.

It should be noted that the physiologic dead space is the volume of gas in a tidal volume that does not participate in gas exchange and is consisted of alveolar and anatomical dead spaces, where the alveolar dead space is the part of gas which enters the alveoli but does not participate in gas exchange due to uneven distribution of blood flows in the lungs, and the anatomical dead space is the volume of gas existing in the airways above the terminal bronchioles, that is, the part of gas in the tidal volume that is exhaled without gas exchange at the beginning of expiration.

In the embodiment of the disclosure, the calculation of the dead space ratio $V_D/V_T$ is as shown in Equation (3)

$$V_D/V_T = (P_{aco_2} - P_{eco_2})/P_{aco_2} \qquad (3)$$

where $P_{aco_2}$ represents the partial pressure value of carbon dioxide within the arterial blood, and $P_{eco_2}$ represents the partial pressure value of carbon dioxide in the expired air. A blood draw is performed on the patient every time the ventilation control module in the apparatus for determining positive end expiratory pressure changes the PEEP value, and a blood gas analyzer is used to measure the $P_{aco_2}$ corresponding to the PEEP value. A carbon dioxide sensor measures the partial pressure value of carbon dioxide in an expiratory phase every time the ventilation control module in the apparatus for determining positive end expiratory pressure changes the PEEP value.

In the embodiment of the disclosure, the physiological parameter monitoring module in the apparatus for determining positive end expiratory pressure may measure an arterial oxygen partial pressure value and an inspired oxygen concentration value corresponding to the PEEP value; and an oxygenation index value corresponding to the PEEP value is calculated according to the arterial oxygen partial pressure value and the inspired oxygen concentration value.

In the embodiment of the disclosure, the oxygenation index $P_{ao_2}/F_{io_2}$ is a quotient value of an arterial oxygen partial pressure $P_{ao_2}$ and an inspired oxygen concentration $F_{io_2}$. The acquisition process of $P_{ao_2}$ is the same as that of $P_{aco_2}$, and they are both measured by the blood gas analyzer under the condition that a blood draw is performed on the patient every time the ventilation control module in the apparatus for determining positive end expiratory pressure changes the PEEP; and clinically, the ventilation device may provide $F_{io_2}$.

At S 103, a change in the at least one physiological parameter is calculated every time the PEEP value changes.

After the physiological parameter monitoring module of the apparatus for determining positive end expiratory pressure detects at least one physiological parameter of the patient, the calculation module of the apparatus for determining positive end expiratory pressure may calculate the change in the at least one physiological parameter every time the PEEP value changes.

In the embodiment of the disclosure, after the physiological parameter monitoring module of the apparatus for determining positive end expiratory pressure monitors the driving pressure value corresponding to the PEEP value, the calculation module of the apparatus for determining positive end expiratory pressure calculates a change in the driving pressure value every time the PEEP value changes.

As an example, as shown in FIG. 2, an airway pressure change curve for 5 respiratory cycles is shown in a first coordinate system, and an airway flow rate change curve for 5 respiratory cycles is shown in a second coordinate system, where each respiratory cycle includes an expiratory phase and an inspiratory phase. In the first coordinate system, an ascending curve represents the inspiratory phase, and a descending curve and a horizontal line together form the expiratory phase. In each expiratory phase, an airway pressure value corresponding to a first horizontal line is Pplat, and an airway pressure value corresponding to a second horizontal line is PEEP, wherein the PEEP is 0 $cmH_2O$ and the Pplat is 15 $cmH_2O$ in a first respiratory cycle, the PEEP is 2 $cmH_2O$ and the Pplat is 15 $cmH_2O$ in a second respiratory cycle, the PEEP is 4 $cmH_2O$ and the Pplat is 16 $cmH_2O$ in a third respiratory cycle, and the PEEP is 6 $cmH_2O$ and the Pplat is 18 $cmH_2O$ in a fourth respiratory cycle, and the PEEP is 8 $cmH_2O$ and the Pplat is 21 $cmH_2O$ in a fifth respiratory cycle, so that the calculation module of the apparatus for determining positive end expiratory pressure can calculate the change in the driving pressure corresponding to each respiratory cycle according to the PEEP and the Pplat in the respiratory cycle.

In the embodiment of the disclosure, after the physiological parameter monitoring module of the apparatus for determining positive end expiratory pressure monitors the compliance value corresponding to the PEEP value, the calculation module of the apparatus for determining positive end expiratory pressure may calculate a change in the compliance value every time the PEEP value changes.

As an example, as shown in FIG. 2, the x-coordinate represents time and the y-coordinate represents an airway flow rate in the second coordinate system, where a positive airway flow rate refers to the inspiratory phase, a negative airway flow rate refers to the expiratory phase, and the tidal volume is a product of multiplying an inspiratory duration in the respiratory cycle by a corresponding inspiratory flow rate. The calculation module of the apparatus for determining positive end expiratory pressure can calculate the change in the compliance corresponding to each respiratory cycle according to the PEEP, the Pplat and the tidal volume values in each respiratory cycle.

In the embodiment of the disclosure, after the physiological parameter monitoring module of the apparatus for determining positive end expiratory pressure monitors the dead space ratio value corresponding to the PEEP value, the calculation module of the apparatus for determining positive end expiratory pressure may calculate a change in the dead space ratio value every time the PEEP value changes.

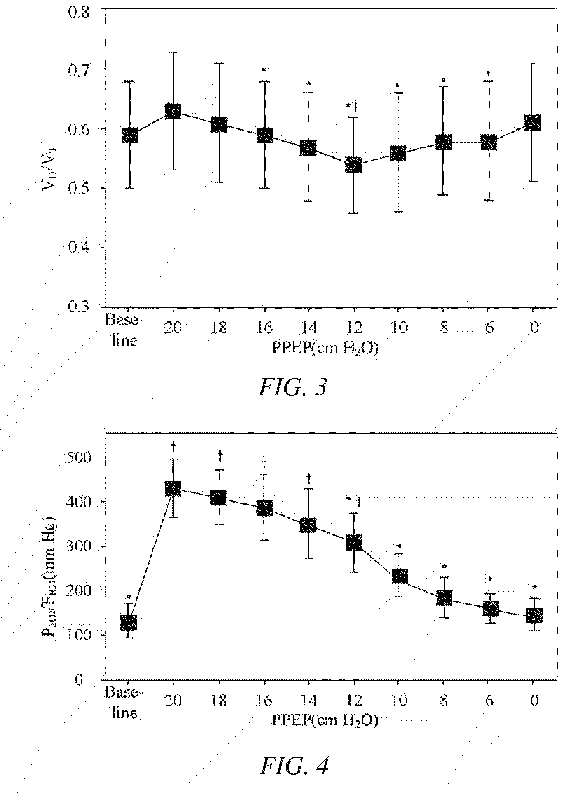

As an example, $V_D/V_T$ values corresponding to the PEEP values of 20 $cmH_2O$, 18 $cmH_2O$, 16 $cmH_2O$, 14 $cmH_2O$, 12 $cmH_2O$, 10 $cmH_2O$, 8 $cmH_2O$, 6 $cmH_2O$, and 0 $cmH_2O$ are shown in the coordinate system of FIG. 3, and the $V_D/V_T$ value shows a descending trend as the PEEP value drops from 20 $cmH_2O$ to 18 $cmH_2O$; the $V_D/V_T$ value shows a descending trend as the PEEP value drops from 18 $cmH_2O$ to 16 $cmH_2O$; the $V_D/V_T$ value shows a descending trend as the PEEP value drops from 16 $cmH_2O$ to 14 $cmH_2O$; the $V_D/V_T$ value shows a descending trend as the PEEP value drops from 14 $cmH_2O$ to 12 $cmH_2O$; the $V_D/V_T$ value shows an ascending trend as the PEEP value drops from 12 $cmH_2O$ to 10 $cmH_2O$; the $V_D/V_T$ value shows an ascending trend as the PEEP value drops from 10 $cmH_2O$ to 8 $cmH_2O$; the $V_D/V_T$ value is unchanged as the PEEP value drops from 8 $cmH_2O$ to 6 $cmH_2O$; and the $V_D/V_T$ value shows an ascending trend as the PEEP value drops from 6 $cmH_2O$ to 0 $cmH_2O$.

In the embodiment of the disclosure, after the physiological parameter monitoring module of the apparatus for determining positive end expiratory pressure monitors the oxygenation index value corresponding to the PEEP value, the calculation module of the apparatus for determining positive end expiratory pressure calculates a rate of change of the oxygenation index value every time the PEEP changes.

As an example, $P_{ao_2}/F_{io_2}$ values corresponding to the PEEP values of 20 $cmH_2O$, 18 $cmH_2O$, 16 $cmH_2O$, 14

$cmH_2O$, 12 $cmH_2O$, 10 $cmH_2O$, 8 $cmH_2O$, 6 $cmH_2O$, and 0 $cmH_2O$ are shown in the coordinate system of FIG. 4, and the calculation module of the apparatus for determining positive end expiratory pressure calculates the rates of change of $P_{ao_2}/F_{io_2}$ respectively, and determines the change in the rates of change of $P_{ao_2}/F_{io_2}$.

At S104, a preferred PEEP value for ventilation to the patient is determined according to the change in the at least one physiological parameter.

After calculating the change in the at least one physiological parameter every time the PEEP value changes, the calculation module of the apparatus for determining positive end expiratory pressure may determine the preferred PEEP value for ventilation to the patient according to the change in the at least one physiological parameter.

In the embodiment of the disclosure, after calculating the change in the compliance value every time the PEEP value changes, the calculation module of the apparatus for determining positive end expiratory pressure may determine the PEEP value corresponding to the maximum value of the compliance as the preferred PEEP value.

Further, after the physiological parameter monitoring module of the apparatus for determining positive end expiratory pressure measures the Pplat value corresponding to the PEEP value, the calculation module of the apparatus for determining positive end expiratory pressure may calculate change values of the PEEP and the Pplat, compares the change value of the PEEP with the change value of the Pplat, and determines the PEEP value as the preferred PEEP when the change value of the PEEP is equal to the change value of the Pplat.

As an example, as shown in FIG. 2, the PEEP is 0 $cmH_2O$ and the Pplat is 15 $cmH_2O$ in the first respiratory cycle, the PEEP is 2 $cmH_2O$ and the Pplat is 15 $cmH_2O$ in the second respiratory cycle; the PEEP is 4 $cmH_2O$ and the Pplat is 16 $cmH_2O$ in the third respiratory cycle; the PEEP is 6 $cmH_2O$ and the Pplat is 18 $cmH_2O$ in the fourth respiratory cycle; and the PEEP is 8 $cmH_2O$ and the Pplat is 21 $cmH_2O$ in the fifth respiratory cycle. It can be calculated that $\Delta$PEEP is 2 and $\Delta$Pplat is 0 in the second respiratory cycle, and $\Delta$PEEP > $\Delta$Pplat for the PEEP of 2 $cmH_2O$; $\Delta$PEEP is 2 and $\Delta$Pplat is 1 in the third respiratory cycle, and $\Delta$PEEP > $\Delta$Pplat for the PEEP of 4 $cmH_2O$; $\Delta$PEEP is 2 and $\Delta$Pplat is 2 in the fourth respiratory cycle, and $\triangle$PEEP = $\triangle$Pplat for the PEEP of 6 $cmH_2O$; and $\triangle$PEEP is 2 and $\triangle$Pplat is 3 in the fifth respiratory cycle, and $\Delta$PEEP < $\Delta$Pplat for the PEEP of 8 $cmH_2O$. The calculation module of the apparatus for determining positive end expiratory pressure then determines the PEEP of 6 $cmH_2O$ as the preferred PEEP value.

It should be noted that if a rising value of the Pplat is less than that of the PEEP every time the ventilation control module of the apparatus for determining positive end expiratory pressure sets the PEEP to a raised value, it is indicated that the compliance of the patient's respiratory system is improved after increasing the PEEP, and the effect of the PEEP is beneficial at the moment; if the rising value of the Pplat is equal to that of the PEEP, it is indicated that the compliance of the patient's respiratory system is not improved after increasing the PEEP; and if the rising value of the Pplat is greater than that of the PEEP, it is indicated that the compliance of the patient's respiratory system decreases after increasing the PEEP, and the PEEP value is too high at the moment.

In the embodiment of the disclosure, after calculating the change in the dead space ratio value every time the PEEP value changes, the calculation module of the apparatus for determining positive end expiratory pressure determines the PEEP value corresponding to the minimum value of the dead space ratio as the preferred PEEP value.

As an example, as shown in FIG. 3, when the PEEP is 12 $cmH_2O$, the $V_D/V_T$ value is minimal. In this case, the ventilation control module of the apparatus for determining positive end expiratory pressure sets the PEEP as 12 $cmH_2O$ to mechanically ventilate the patient.

In the embodiment of the disclosure, after calculating the rate of change of the oxygenation index corresponding to each PEEP value, the calculation module of the apparatus for determining positive end expiratory pressure may determine the PEEP value corresponding to the maximum point of the rate of change of the oxygenation index as the preferred PEEP value.

As an example, as shown in FIG. 4, when the PEEP is 12 $cmH_2O$, the rate of change of the $P_{ao_2}/F_{io_2}$ value is maximal. In this case, the ventilation control module of the apparatus for determining positive end expiratory pressure sets the PEEP as 12 $cmH_2O$ to mechanically ventilate the patient.

Further, the calculation module of the apparatus for determining positive end expiratory pressure determines respective candidate PEEP values according to the changes in the respective physiological parameters, and then determines the preferred PEEP value for ventilation to the patient according to the candidate PEEP values.

Specifically, after determining the PEEP value corresponding to the minimum value of the driving pressure, the PEEP value corresponding to the maximum value of the compliance, the PEEP value corresponding to the minimum value of the dead space ratio and the PEEP value corresponding to the maximum point of the rate of change of the oxygenation index, the calculation module of the apparatus for determining positive end expiratory pressure may obtain any one of the PEEP value corresponding to the minimum value of the driving pressure and the PEEP value corresponding to the maximum value of the compliance; and perform average calculation on at least two of any one of the PEEP values corresponding to the minimum value of the driving pressure and the maximum value of the compliance, the PEEP value corresponding to the minimum value of the dead space ratio and the PEEP value corresponding to the maximum point of the rate of change of the oxygenation index, where the average value is determined as the preferred PEEP value.

Optionally, the calculation module of the apparatus for determining positive end expiratory pressure may perform average calculation on the PEEP value corresponding to the minimum value of the dead space ratio and any one of the PEEP values corresponding to the minimum value of the driving pressure and the maximum value of the compliance; and the average value is determined as the preferred PEEP value.

Optionally, the calculation module of the apparatus for determining positive end expiratory pressure may perform average calculation on the PEEP value corresponding to the maximum point of the rate of change of the oxygenation index and any one of the PEEP values corresponding to the minimum value of the driving pressure and the maximum value of the compliance; and the average value is determined as the preferred PEEP value.

Optionally, the calculation module of the apparatus for determining positive end expiratory pressure may perform average calculation on the PEEP value corresponding to the minimum value of the dead space ratio and the PEEP value corresponding to the maximum point of the rate of change of the oxygenation index and any one of the PEEP values corresponding to the minimum value of the driving pressure and the maximum value of the compliance; and the average value is determined as the preferred PEEP value.

Further, when the calculation module of the apparatus for determining positive end expiratory pressure determines the preferred PEEP value for ventilation to the patient, the output module of the apparatus for determining positive end expiratory pressure outputs the preferred PEEP value according to a preset prompting strategy, so as to prompt the doctor that the preferred PEEP value can be used for ventilation.

It can be understood that the apparatus for determining positive end expiratory pressure stepwise changes the PEEP value, calculates the change in the at least one physiological parameter every time the PEEP value changes, and determines the preferred PEEP value for ventilation to the patient according to the change in the at least one physiological parameter. In this case, the apparatus for determining positive end expiratory pressure can determine different preferred PEEP values according to the physiological parameters of different patients to conduct mechanical ventilation, and can automatically determine preferred PEEP values.

The process in which the apparatus for determining positive end expiratory pressure determines the preferred PEEP value according to the changes in different parameter values will be embodied in the following embodiments.

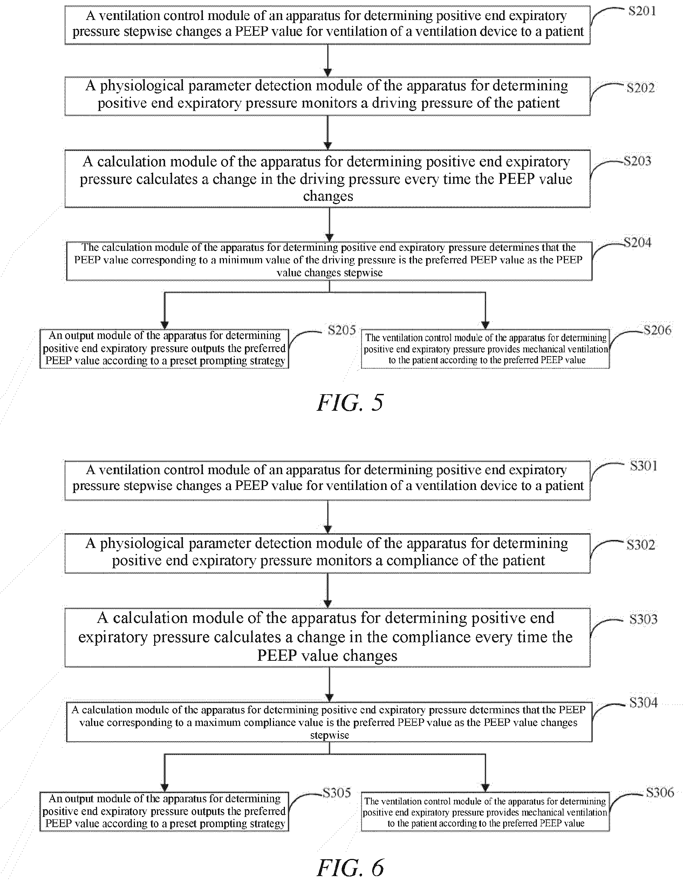

Another embodiment of the disclosure provides a method for determining positive end expiratory pressure, which is applicable to a ventilation device. The ventilation device includes an apparatus for determining positive end expiratory pressure, where the apparatus for determining positive end expiratory pressure include a ventilation control module, a physiological parameter monitoring module, a calculation module and an output module. The method for determining positive end expiratory pressure provided by the embodiment of the disclosure is applicable to a situation where the ventilation device determines a preferred PEEP value according to a driving pressure parameter of a patient. As shown in FIG. 5, the method may include the following steps.

At S201, the ventilation control module of the apparatus for determining positive end expiratory pressure stepwise changes a PEEP value for ventilation of the ventilation device to a patient.

The description of step S201 of the embodiment of this disclosure here is consistent with the description of step S 101 in the first embodiment and will not be repeated herein.

At S202, the physiological parameter monitoring module of the apparatus for determining positive end expiratory pressure monitors the driving pressure of the patient.

After the ventilation control module of the apparatus for determining positive end expiratory pressure stepwise changes the PEEP value for ventilation of the ventilation device to the patient, the physiological parameter monitoring module of the apparatus for determining positive end expiratory pressure monitors the driving pressure of the patient.

In the embodiment of the disclosure, the physiological parameter monitoring module in the apparatus for determining positive end expiratory pressure measures an airway plateau pressure value corresponding to the PEEP value; and the driving pressure value is calculated according to the PEEP value and the airway plateau pressure value. Specifically, the calculation of the driving pressure value according to the PEEP value and the airway plateau pressure value is as shown in Equation (1),

$$\Delta P = Pplat - PEEP \qquad (1)$$

where $\Delta P$ represents the driving pressure value, and Pplat represents the airway plateau pressure value.

At S203, the calculation module of the apparatus for determining positive end expiratory pressure calculates a change in the driving pressure every time the PEEP value changes.

After the physiological parameter monitoring module of the apparatus for determining positive end expiratory pressure monitors the driving pressure of the patient, the calculation module of the apparatus for determining positive end expiratory pressure calculates the change in the driving pressure every time the PEEP value changes.

In the embodiment of the disclosure, as shown in FIG. 2, an airway pressure change curve for 5 respiratory cycles is shown in a first coordinate system, and an airway flow rate change curve for 5 respiratory cycles is shown in a second coordinate system, where each respiratory cycle includes an expiratory phase and an inspiratory phase. In the first coordinate system, an ascending curve represents the inspiratory phase, and a descending curve and a horizontal line together form the expiratory phase. In each expiratory phase, an airway pressure value corresponding to a first horizontal line is Pplat, and an airway pressure value corresponding to a second horizontal line is PEEP, where the PEEP is 0 cmH$_2$O and the Pplat is 15 cmH$_2$O in a first respiratory cycle, the PEEP is 2 cmH$_2$O and the Pplat is 15 cmH$_2$O in a second respiratory cycle, the PEEP is 4 cmH$_2$O and the Pplat is 16 cmH$_2$O in a third respiratory cycle, and the PEEP is 6 cmH$_2$O and the Pplat is 18 cmH$_2$O in a fourth respiratory cycle, and the PEEP is 8 cmH$_2$O and the Pplat is 21 cmH$_2$O in a fifth respiratory cycle, so that the calculation module of the apparatus for determining positive end expiratory pressure can calculate the change in the driving pressure corresponding to each respiratory cycle according to the PEEP and the Pplat in each respiratory cycle.

At S204, the calculation module of the apparatus for determining positive end expiratory pressure determines that the PEEP value corresponding to the minimum value of the driving pressure is the preferred PEEP value as the PEEP value changes stepwise.

After calculating the change in the driving pressure every time the PEEP value changes, the calculation module of the apparatus for determining positive end expiratory pressure determines that the PEEP value corresponding to the minimum value of the driving pressure is the preferred PEEP value as the PEEP value changes stepwise.

In the embodiment of the disclosure, the apparatus for determining positive end expiratory pressure finds out the PEEP value corresponding to the minimum driving pressure from the PEEP values in different phases, and determines the PEEP value corresponding to the minimum driving pressure as the preferred PEEP value.

At S205, the output module of the apparatus for determining positive end expiratory pressure outputs the preferred PEEP value according to a preset prompting strategy.

After the calculation module of the apparatus for determining positive end expiratory pressure determines that the PEEP value corresponding to the minimum value of the driving pressure is the preferred PEEP value as the PEEP value changes stepwise, the output module of the apparatus for determining positive end expiratory pressure outputs the preferred PEEP value according to the preset prompting strategy.

In the embodiment of the disclosure, the output module of the apparatus for determining positive end expiratory pressure outputs the preferred PEEP value according to the preset prompting strategy when the calculation module of the apparatus for determining positive end expiratory pressure calculates the preferred PEEP value.

Optionally, the output module of the apparatus for determining positive end expiratory pressure prompts a doctor about the preferred PEEP value. In this case, the doctor can control the ventilation control module to provide mechanical ventilation to the patient using the PEEP value.

Optionally, the preset prompting strategy may be a voice prompt or prompt information displayed on a display interface or in other forms, and is specifically selected according to the actual situation, which is not specifically limited in the embodiment of the disclosure.

At S206, the ventilation control module of the apparatus for determining positive end expiratory pressure provides the mechanical ventilation to the patient according to the preferred PEEP value.

After the calculation module of the apparatus for determining positive end expiratory pressure determines that the PEEP value corresponding to the minimum value of the driving pressure is the preferred PEEP value as the PEEP value changes stepwise, the ventilation control module of the apparatus for determining positive end expiratory pressure provides the mechanical ventilation to the patient according to the preferred PEEP value.

In the embodiment of the disclosure, the ventilation control module of the apparatus for determining positive end expiratory pressure provides the mechanical ventilation to the patient using the preferred PEEP value.

S205 and S206 can be two parallel steps after S204 and are specifically executed selectively according to the actual situation, which is not specifically limited in the embodiment of the disclosure.

Another embodiment of the disclosure provides a method for determining positive end expiratory pressure, which is applicable to a ventilation device. The ventilation device can include an apparatus for determining positive end expiratory pressure, where the apparatus for determining positive end expiratory pressure may include a ventilation control module, a physiological parameter monitirong module, a calculation module and an output module. The method for determining positive end expiratory pressure provided by the embodiment of the disclosure is applicable to a situation where the ventilation device determines a preferred PEEP value according to a compliance parameter of a patient. As shown in FIG. 6, the method may include the following steps.

At S301, the ventilation control module of the apparatus for determining positive end expiratory pressure stepwise changes a PEEP value for ventilation of the ventilation device to a patient.

The description of step S301 of the embodiment of this disclosure here is consistent with the description of step S201 in the second embodiment, and will not be repeated herein.

At S302, the physiological parameter monitoring module of the apparatus for determining positive end expiratory pressure monitors a compliance of the patient.

After the ventilation control module of the apparatus for determining positive end expiratory pressure stepwise changes the PEEP value for ventilation of the ventilation device to the patient, the physiological parameter monitoring module of the apparatus for determining positive end expiratory pressure may monitor the compliance of the patient.

In the embodiment of the disclosure, the physiological parameter monitoring module in the apparatus for determining positive end expiratory pressure can calculate a tidal volume according to an inspiratory duration and an inspiratory flow rate; and a compliance value is calculated according to the tidal volume, a PEEP value and an airway plateau pressure value, where the calculation of the compliance value is as shown in Equation (2),

$$ \text{Compliance} \ = \ \text{Tidal volume} \ / \ (\text{Pplat} - \text{PEEP}) \qquad (2) $$

At S303, the calculation module of the apparatus for determining positive end expiratory pressure calculates a change in the compliance every time the PEEP value changes.

After the physiological parameter monitoring module of the apparatus for determining positive end expiratory pressure monitors the compliance of the patient, the calculation module of the apparatus for determining positive end expiratory pressure may calculate the change in the compliance every time the PEEP value changes.

In the embodiment of the disclosure, as shown in FIG. 2, the x-coordinate represents time and the y-coordinate represents an airway flow rate in the second coordinate system, where a positive airway flow rate refers to the inspiratory phase, a negative airway flow rate refers to the expiratory phase, and the tidal volume is a product of multiplying an inspiratory duration in the respiratory cycle by a corresponding inspiratory flow rate. The calculation module of the apparatus for determining positive end expiratory pressure can calculate the change in the compliance corresponding to each respiratory cycle according to the PEEP, the Pplat and the tidal volume values in each respiratory cycle.

At S304, the calculation module of the apparatus for determining positive end expiratory pressure determines that the PEEP value corresponding to the maximum value of the compliance is the preferred PEEP value as the PEEP value changes stepwise.

After calculating the change in the compliance every time the PEEP value changes, the calculation module of the apparatus for determining positive end expiratory pressure determines that the PEEP value corresponding to the maximum value of the compliance is the preferred PEEP value as the PEEP value changes stepwise.

In the embodiment of the disclosure, the apparatus for determining positive end expiratory pressure finds out the PEEP value corresponding to the maximum compliance from the PEEP values in different phases, and determines the PEEP value corresponding to the maximum compliance as the preferred PEEP value.

At S305, the output module of the apparatus for determining positive end expiratory pressure outputs the preferred PEEP value according to a preset prompting strategy.

The description of step S305 of the embodiment of this disclosure here is consistent with the description of step S205 in the second embodiment, and will not be repeated herein.

At S306, the ventilation control module of the apparatus for determining positive end expiratory pressure provides the mechanical ventilation to the patient according to the preferred PEEP value.

The description of step S306 of the embodiment of this disclosure here is consistent with the description of step S206 in the second embodiment, and will not be repeated herein.

S305 and S306 can be two parallel steps after S304 and are specifically executed selectively according to the actual situation, which is not specifically limited in the embodiment of the disclosure.

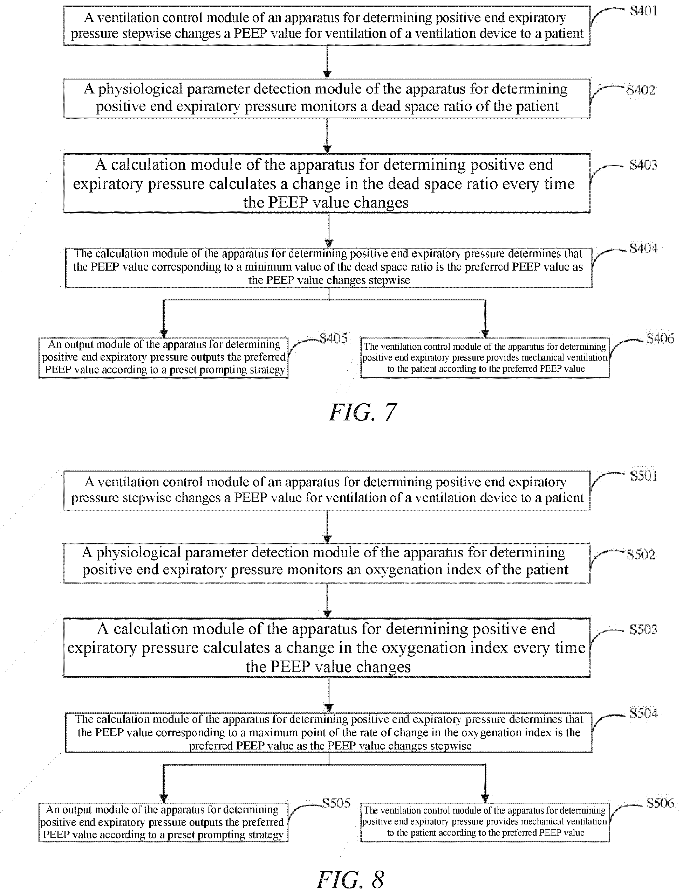

A yet another embodiment of the disclosure provides a method for determining positive end expiratory pressure, which is applicable to a ventilation device. The ventilation device can include an apparatus for determining positive end expiratory pressure, where the apparatus for determining positive end expiratory pressure may include a ventilation control module, a physiological parameter monitoring module, a calculation module and an output module. The method for determining positive end expiratory pressure provided by the embodiment of the disclosure is applicable to a situation where the ventilation device determines a preferred PEEP value according to a dead space ratio parameter of a patient. As shown in FIG. 7, the method may include the following steps.

At S40 1, the ventilation control module of the apparatus for determining positive end expiratory pressure stepwise changes a PEEP value for ventilation of the ventilation device to a patient.

The description of step S401 of the embodiment of this disclosure here is consistent with the description of step S201 in the second embodiment, and will not be repeated herein.

At S402, the physiological parameter monitoring module of the apparatus for determining positive end expiratory pressure can monitor a dead space ratio of the patient.

After the ventilation control module of the apparatus for determining positive end expiratory pressure stepwise changes the PEEP value for ventilation of the ventilation device to the patient, the physiological parameter monitoring module of the apparatus for determining positive end expiratory pressure may monitor the dead space ratio of the patient.

In the embodiment of the disclosure, the physiological parameter monitoring module in the apparatus for determining positive end expiratory pressure measures a partial pressure value of carbon dioxide within the arterial blood corresponding to the PEEP value and a partial pressure value of carbon dioxide in the expired air corresponding to the PEEP value; and a dead space ratio value corresponding to the PEEP value is calculated according to the partial pressure value of carbon dioxide within the arterial blood and the partial pressure value of carbon dioxide in the expired air.

It should be noted that the physiologic dead space is the volume of gas in a tidal volume that does not participate in gas exchange and is consisted of alveolar and anatomical dead spaces, where the alveolar dead space is the part of gas which enters the alveoli but does not participate in gas exchange due to uneven distribution of blood flows in the lungs, and the anatomical dead space is the volume of gas existing in the airways above the terminal bronchioles, that is, the part of gas in the tidal volume that is exhaled without gas exchange at the beginning of expiration.

In the embodiment of the disclosure, the calculation of the dead space ratio $V_D/V_T$ is as shown in Equation (3)

$$V_D/V_T = (P_{aco_2} - P_{eco_2})/P_{aco_2} \qquad (3)$$

where $P_{aco2}$ represents the partial pressure value of carbon dioxide within the arterial blood, and $P_{eco_2}$ represents the partial pressure value of carbon dioxide in the expired air. A blood draw is performed on the patient every time the ventilation control module in the apparatus for determining positive end expiratory pressure changes the PEEP value, and a blood gas analyzer is used to measure the $P_{aco_2}$ corresponding to the PEEP value. A carbon dioxide sensor measures the partial pressure value of carbon dioxide in an expiratory phase every time the ventilation control module in the apparatus for determining positive end expiratory pressure changes the PEEP value.

At S403, the calculation module of the apparatus for determining positive end expiratory pressure calculates a change in the dead space ratio every time the PEEP value changes.

After the physiological parameter monitoring module of the apparatus for determining positive end expiratory pressure monitors the dead space ratio of the patient, the calculation module of the apparatus for determining positive end expiratory pressure may calculate the change in the dead space ratio every time the PEEP value changes.

In the embodiment of the disclosure, $V_D/V_T$ values corresponding to the PEEP values of 20 cmH$_2$O, 18 cmH$_2$O, 16 cmH$_2$O, 14 cmH$_2$O, 12 cmH$_2$O, 10 cmH$_2$O, 8 cmH$_2$O, 6 cmH$_2$O, and 0 cmH$_2$O are shown in the coordinate system of FIG. 3. Here, the $V_D/V_T$ value shows a descending trend as the PEEP value drops from 20 cmH$_2$O to 18 cmH$_2$O; the $V_D/V_T$ value shows a descending trend as the PEEP value drops from 18 cmH$_2$O to 16 cmH$_2$O; the $V_D/V_T$ value shows a descending trend as the PEEP value drops from 16 cmH$_2$O to 14 cmH$_2$O; the $V_D/V_T$ value shows a descending trend as the PEEP value drops from 14 cmH$_2$O to 12 cmH$_2$O; the $V_D/V_T$ value shows an ascending trend as the PEEP value drops from 12 cmH$_2$O to 10 cmH$_2$O; the $V_D/V_T$ value shows an ascending trend as the PEEP value drops from 10 cmH$_2$O to 8 cmH$_2$O; the $V_D/V_T$ value is unchanged as the PEEP value drops from 8 cmH$_2$O to 6 cmH$_2$O; and the $V_D/V_T$ value shows an ascending trend as the PEEP value drops from 6 cmH$_2$O to 0 cmH$_2$O.

At S404, the calculation module of the apparatus for determining positive end expiratory pressure determines that the PEEP value corresponding to the minimum value of the dead space ratio is the preferred PEEP value as the PEEP value changes stepwise.

After calculating the change in the dead space ratio every time the PEEP value changes, the calculation module of the apparatus for determining positive end expiratory pressure determines that the PEEP value corresponding to the minimum value of the dead space ratio is the preferred PEEP value as the PEEP value changes stepwise.

In the embodiment of the disclosure, the apparatus for determining positive end expiratory pressure finds out the PEEP value corresponding to the minimum value of the dead space ratio from the PEEP values in different phases, and determines the PEEP value corresponding to the minimum value of the dead space ratio as the preferred PEEP value.

At S405, the output module of the apparatus for determining positive end expiratory pressure outputs the preferred PEEP value according to a preset prompting strategy.

The description of step S405 of the embodiment of this disclosure here is consistent with the description of step S205 in the second embodiment, and will not be repeated herein.

At S406, the ventilation control module of the apparatus for determining positive end expiratory pressure provides the mechanical ventilation to the patient according to the preferred PEEP value.

The description of step S406 of the embodiment of this disclosure here is consistent with the description of step S206 in the second embodiment, and will not be repeated herein.

S405 and S406 can be two parallel steps after S404 and are specifically executed selectively according to the actual situation, which is not specifically limited in the embodiment of the disclosure.

Another embodiment of the disclosure provides a method for determining positive end expiratory pressure, which is applicable to a ventilation device. The ventilation device can include an apparatus for determining positive end expiratory pressure, where the apparatus for determining positive end expiratory pressure may include a ventilation control module, a physiological parameter monitoring module, a calculation module and an output module. The method for determining positive end expiratory pressure provided by the embodiment of the disclosure is applicable to a situation where the ventilation device determines a preferred PEEP value according to an oxygenation index parameter of a patient. As shown in FIG. 8, the method may include the following steps.

At S501, the ventilation control module of the apparatus for determining positive end expiratory pressure stepwise changes a PEEP value for ventilation of the ventilation device to a patient.

The description of step S501 of the embodiment of this disclosure here is consistent with the description of step S201 in the second embodiment, and will not be repeated herein.

At S502, the physiological parameter monitoring module of the apparatus for determining positive end expiratory pressure monitors an oxygenation index of the patient.

After the ventilation control module of the apparatus for determining positive end expiratory pressure stepwise changes the PEEP value for ventilation of the ventilation device to the patient, the physiological parameter monitoring module of the apparatus for determining positive end expiratory pressure may monitor the oxygenation index of the patient.

In the embodiment of the disclosure, the physiological parameter monitoring module in the apparatus for determining positive end expiratory pressure measures an arterial oxygen partial pressure value corresponding to the PEEP value and an inspired oxygen concentration value corresponding to the PEEP value; and an oxygenation index value corresponding to the PEEP value is calculated according to the arterial oxygen partial pressure value and the inspired oxygen concentration value.

In the embodiment of the disclosure, the oxygenation index $P_{ao_2}/F_{io_2}$ is a quotient value of an arterial oxygen partial pressure $P_{ao_2}$ and an inspired oxygen concentration $F_{io_2}$, where the acquisition process of $P_{ao_2}$ is the same as that of $P_{aco_2}$, and they are both measured by the blood gas analyzer under the condition that a blood draw is performed on the patient every time the ventilation control module in the apparatus for determining positive end expiratory pressure changes the PEEP; and clinically, the ventilation device may provide $F_{io_2}$.

At S503, the calculation module of the apparatus for determining positive end expiratory pressure calculates a change in the oxygenation index every time the PEEP value changes.

After the physiological parameter monitoring module of the apparatus for determining positive end expiratory pressure monitors the oxygenation index of the patient, the calculation module of the apparatus for determining positive end expiratory pressure may calculate the change in the oxygenation index every time the PEEP value changes.

In the embodiment of disclosure, $P_{ao_2}/F_{io_2}$ values corresponding to the PEEP values of 20 $cmH_2O$, 18 $cmH_2O$, 16 $cmH_2O$, 14 $cmH_2O$, 12 $cmH_2O$, 10 $cmH_2O$, 8 $cmH_2O$, 6 $cmH_2O$, and 0 $cmH_2O$ are shown in the coordinate system of FIG. 4, and the calculation module of the apparatus for determining positive end expiratory pressure calculates the rates of change of $P_{ao_2}/F_{io_2}$ respectively, and determines the change in the rates of change of $P_{ao_2}/F_{io_2}$.

At S504, the calculation module of the apparatus for determining positive end expiratory pressure determines that the PEEP value corresponding to a maximum point of the rate of change of the oxygenation index is the preferred PEEP value as the PEEP value changes stepwise.

After calculating the change in the oxygenation index every time the PEEP value changes, the calculation module of the apparatus for determining positive end expiratory pressure determines that the PEEP value corresponding to the maximum point of the rate of change of the oxygenation index is the preferred PEEP value as the PEEP value changes stepwise.

In the embodiment of the disclosure, the apparatus for determining positive end expiratory pressure finds out the PEEP value corresponding to the maximum point of the rate of change of the oxygenation index from the PEEP values in different phases, and determines the PEEP value corresponding to the maximum point of the rate of change of the oxygenation index as the preferred PEEP value.

At S505, the output module of the apparatus for determining positive end expiratory pressure outputs the preferred PEEP value according to a preset prompting strategy.

The description of step S505 of the embodiment of this disclosure here is consistent with the description of step S205 in the second embodiment, and will not be repeated herein.

At S506, the ventilation control module of the apparatus for determining positive end expiratory pressure provides the mechanical ventilation to the patient according to the preferred PEEP value.

The description of step S506 of the embodiment of this disclosure here is consistent with the description of step S206 in the second embodiment, and will not be repeated herein.

S505 and S506 can be two parallel steps after S504 and are specifically executed selectively according to the actual situation, which is not specifically limited in the embodiment of the disclosure.

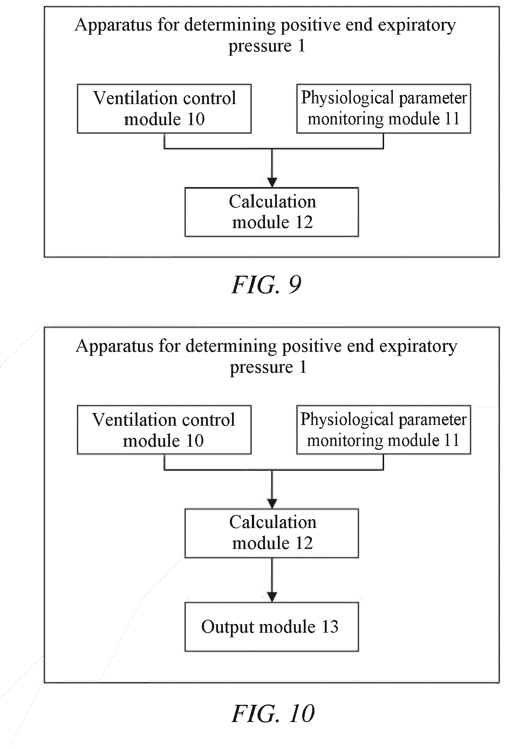

A still further embodiment of the disclosure provides an apparatus for determining positive end expiratory pressure 1. As shown in FIG. 9, the apparatus for determining positive end expiratory pressure 1 may include:

a ventilation control module 10 for stepwise changing a PEEP value for ventilation of a ventilation device to a patient;
a physiological parameter monitoring module 11 for monitoring at least one physiological parameter of the patient; and
a calculation module 12 for calculating a change in the at least one physiological parameter every time the PEEP value changes and determining a preferred PEEP value for ventilation to the patient according to the change in the at least one physiological parameter.

Optionally, the at least one physiological parameter includes a driving pressure. The calculation module 12 determines that the PEEP value corresponding to a minimum value of the driving pressure is the preferred PEEP value as the PEEP value changes stepwise.

Optionally, the at least one physiological parameter includes a compliance. The calculation module 12 determines that the PEEP value corresponding to a maximum value of the compliance is the preferred PEEP value as the PEEP value changes stepwise.

Optionally, the at least one physiological parameter includes a dead space ratio. The calculation module 12 determines that the PEEP value corresponding to a minimum value of the dead space ratio is the preferred PEEP value as the PEEP value changes stepwise.

Optionally, the at least one physiological parameter includes an oxygenation index. The calculation module 12 determines that the PEEP value corresponding to a maximum point of a rate of change of the oxygenation index is the preferred PEEP value as the PEEP value changes stepwise.

Optionally, the at least one physiological parameter includes: at least two of the driving pressure, the compliance, the dead space ratio, and the oxygenation index. In this case, the calculation module 12 determines respective candidate PEEP values according to the changes in the respective at least two physiological parameters, and determines a preferred PEEP value for ventilation to the patient according to the respective determined candidate PEEP values.

Specifically, depending on the changes in the respective physiological parameters, the calculation module 12 may obtain the respective candidate PEEP values determined according to the changes in the respective physiological parameters. For example, a corresponding candidate PEEP value may be obtained according to the change in the driving pressure, and a corresponding candidate PEEP value may be obtained according to the change in the compliance, so that two candidate PEEP values can be obtained. The method of determining the candidate PEEP values according to the changes in the physiological parameters is the same as the above method of determining the preferred PEEP value according to the physiological parameter, and will not be described in detail herein. The calculation module 12 may then determine the preferred PEEP value for ventilation to the patient according to the two candidate PEEP values. In a particular implementation, the calculation module 12 may take an average value of the two candidate PEEP values as the preferred PEEP value, may determine the preferred PEEP value from a plurality of candidate PEEP values according to a preset weight factors and so on, and may also take the two candidate PEEP values as the preferred PEEP values.

Optionally, as shown in FIG. 10, the apparatus further includes: an output module 13.

The output module 13 outputs the preferred PEEP value according to a preset prompting strategy.

In the case where the preferred PEEP values include two or more candidate PEEP values, the output module 13 may simultaneously output a plurality of determined candidate PEEP values for reference by a doctor.

Optionally, the ventilation control module 10 may further provide mechanical ventilation to the patient according to the preferred PEEP value. Of course, if the calculation module 12 obtains two or more candidate PEEP values according to the changes in the respective physiological parameters in the case where mechanical ventilation is needed for a patient based on the preferred PEEP value, the calculation module 12 also needs to determine a preferred PEEP from the plurality of candidate PEEP values according to a prediction strategy. In a particular implementation, an average value of a plurality of candidate PEEP values may be taken as the preferred PEEP value, or the preferred PEEP value may also be determined from a plurality of candidate PEEP values according to a preset weight factors and so on.

An embodiment of the disclosure provides the apparatus for determining positive end expiratory pressure 1, where the apparatus for determining positive end expiratory pressure 1 stepwise changes the PEEP value for ventilation of the ventilation device to the patient, monitors the at least one physiological parameter of the patient, calculates the change in the at least one physiological parameter every time the PEEP value changes, and determines the preferred PEEP value for ventilation to the patient according to the change in the at least one physiological parameter. In other words, the apparatus for determining positive end expiratory pressure 1 provided by the embodiment of the disclosure stepwise changes the PEEP value, calculates the change in the at least one physiological parameter every time the PEEP value changes, and determines the preferred PEEP value for ventilation to the patient according to the change in the at least one physiological parameter. In this case, the apparatus for determining positive end expiratory pressure 1 determines different preferred PEEP values based on the physiological parameters of different patients to conduct mechanical ventilation, and can automatically determine preferred PEEP values.

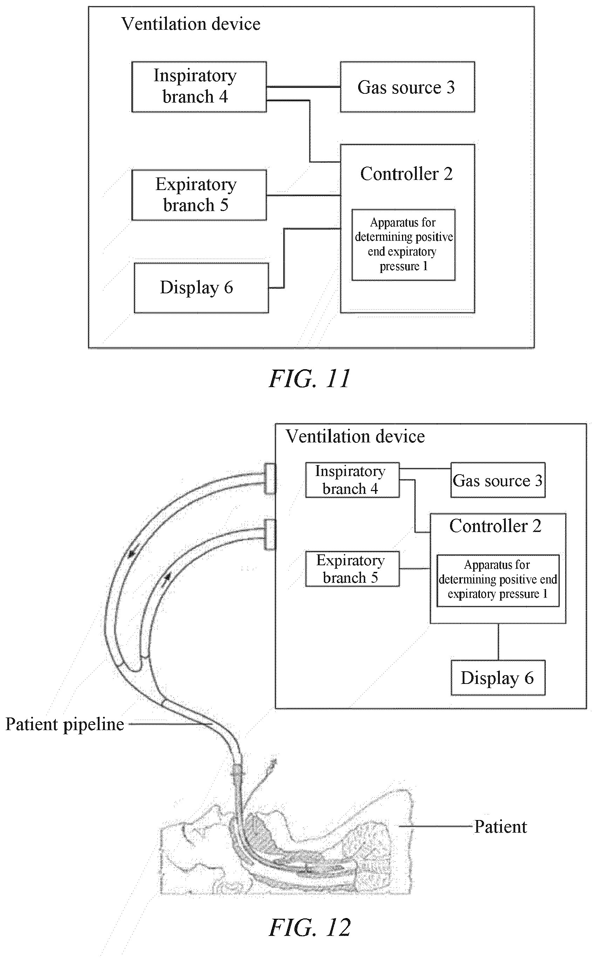

An embodiment of the disclosure further provides a ventilation device. FIG. is first schematic structural diagram of a ventilation device provided by an embodiment of the disclosure. As shown in FIG. 11, the ventilation device includes a controller 2 that includes the apparatus for determining positive end expiratory pressure 1 described above, and the ventilation device further includes: a gas source 3, an inspiratory branch 4, an expiratory branch 5, and a display 6.

The gas source 3 provides a gas during mechanical ventilation.

The inspiratory branch 4 is connected to the gas source 3 and provides an inspiratory path during the mechanical ventilation.

The expiratory branch 5 provides an expiratory path during the mechanical ventilation.

The controller 2 is connected to the inspiratory branch 4 and the expiratory branch 5.

The apparatus for determining positive end expiratory pressure 1 determines a preferred PEEP value during the mechanical ventilation.

The display 5 is connected to the controller 2 and displays a respiratory waveform during the mechanical ventilation.

FIG. 12 is a second schematic structural diagram of a ventilation device provided according to an embodiment of the disclosure. As shown in FIG. 12, a patient can be connected to a ventilation device through a patient pipeline to implement mechanical ventilation, where the ventilation device includes the apparatus for determining positive end expiratory pressure described above.

An embodiment of the disclosure provides a computer-readable storage medium, wherein the computer-readable storage medium stores a positive end expiratory pressure determining program, and the positive end expiratory pressure determining program is executable by a processor to implement the positive end expiratory pressure determining method described above. The computer-readable storage medium may be a volatile memory, such as a Random-Access Memory (RAM), or a non-volatile memory, such as a Read-Only Memory (ROM), a flash memory, a Hard Disk Drive (HDD) or a Solid-State Drive (SSD); or a respective device comprising one or any combination of the above memories, such as a mobile phone, a computer, a tablet, and a personal digital assistant.

It should be noted that the term "comprise", "include", or any other variant thereof herein is intended to encompass a non-exclusive inclusion, such that a process, method, article, or system that includes a series of elements not only comprises those elements, but also comprises other elements not explicitly listed, or elements that are inherent to such a process, method, article, or system. In the absence of more restrictions, the element defined by the phrase "comprising a/an ..." does not exclude the presence of a further identical element in the process, method, article or system that includes the element.

The serial numbers of the embodiments of the disclosure described above are merely for description, and do not represent the superiority of the embodiments.

Through the description of the foregoing embodiments, those skilled in the art can clearly understand that the method of the foregoing embodiments can be implemented by means of software combined with a necessary universal hardware platform, and certainly, can also be implemented through hardware, but in many cases, the former is a better implementation. Based on such an understanding, the technical solution of the disclosure substantively, or the part thereof making a contribution to the prior art may be embodied in the form of a software product. The computer software product is stored in a storage medium (such as an ROM/RAM, a magnetic disk or an optical disc), including a number of instructions for causing a terminal (which may be a mobile phone, a computer, a server, an air conditioner, a network device, etc.) to perform the methods described in the various embodiments of the disclosure.

The embodiments of the disclosure are described above with reference to the accompanying drawings, but the disclosure is not limited to the specific embodiments described above, which are merely illustrative and not restrictive. Those skilled in the art may also make many forms in light of the disclosure without departing from the scope of protection of the claims, and these forms all fall into the scope of protection of the disclosure.

Industrial applicability

In the embodiments of the disclosure, the apparatus for determining positive end expiratory pressure provided by the embodiment of the disclosure stepwise changes the PEEP value, calculates the change in the at least one physiological parameter every time the PEEP value changes, and determines the preferred PEEP value for ventilation to the patient according to the change in the at least one physiological parameter. In this case, the apparatus for determining positive end expiratory pressure can determine different preferred PEEP values based on the physiological parameters of different patients to conduct mechanical ventilation, and can automatically determine preferred PEEP values.

**Claims**

1. A computer program for determining positive end expiratory pressure comprising instructions to cause a ventilation device to execute the steps comprising:

   controlling a ventilation control module (10) for stepwise changing a PEEP value for ventilation of the ventilation device to a patient;
   controlling a physiological parameter monitoring module (11) for monitoring at least one physiological parameter of the patient;
   controlling a calculation module (12) for calculating a change in the at least one physiological parameter every time the PEEP value is changed; and

determining a preferred PEEP value for ventilation to the patient according to the change in the at least one physiological parameter ;

**characterized in that**, the at least one physiological parameter comprises at least two of a driving pressure, a compliance, a dead space ratio and an oxygenation index, and determining a preferred PEEP value for ventilation to the patient according to the change in the at least one physiological parameter comprises: determining respective candidate PEEP values according to the changes in the respective at least two physiological parameters; and determining the preferred PEEP value for ventilation to the patient according to the respective candidate PEEP values;

the candidate PEEP values comprise any one of the PEEP value corresponding to a minimum value of the driving pressure and the PEEP value corresponding to a maximum value of the compliance, and at least one of the PEEP value corresponding to a minimum value of the dead space ratio and the PEEP value corresponding to a maximum point of a change rate of the oxygenation index;

wherein determining the preferred PEEP value for ventilation to the patient according to the respective candidate PEEP values comprises:

determining the preferred PEEP value according to the PEEP value corresponding to the minimum value of the dead space ratio and any one of the PEEP value corresponding to the minimum value of the driving pressure and the PEEP value corresponding to the maximum value of the compliance; or, determining the preferred PEEP value according to the PEEP value corresponding to the maximum point of the change rate of the oxygenation index and any one of the PEEP value corresponding to the minimum value of the driving pressure and the PEEP value corresponding to the maximum value of the compliance; or, determining the preferred PEEP value according to the PEEP value corresponding to the minimum value of the dead space ratio, the PEEP value corresponding to the maximum point of the change rate of the oxygenation index and any one of the PEEP value corresponding to the minimum value of the driving pressure and the PEEP value corresponding to the maximum value of the compliance.

2. The computer program of claim 1, **characterized in that** the at least one physiological parameter is the driving pressure; and

determining a preferred PEEP value for ventilation to the patient according to the change in the at least one physiological parameter comprises:

setting the PEEP value corresponding to the minimum value of the driving pressure to be the preferred PEEP value as the PEEP value changes stepwise.

3. The computer program of claim 1, **characterized in that** the at least one physiological parameter is the compliance; and

determining a preferred PEEP value for ventilation to the patient according to the change in the at least one physiological parameter comprises:

setting the PEEP value corresponding to the maximum value of the compliance to be the preferred PEEP value as the PEEP value changes stepwise.

4. The computer program of claim 1, **characterized in that** the at least one physiological parameter is the dead space ratio; and

determining a preferred PEEP value for ventilation to the patient according to the change in the at least one physiological parameter comprises:

setting the PEEP value corresponding to the minimum value of the dead space ratio to be the preferred PEEP value as the PEEP value changes stepwise.

5. The computer program of claim 1, **characterized in that** the at least one physiological parameter is the oxygenation index; and

determining a preferred PEEP value for ventilation to the patient according to the change in the at least one physiological parameter comprises:

setting the PEEP value corresponding to the maximum point of the change rate of the oxygenation index to be the preferred PEEP value as the PEEP value changes stepwise.

6. The computer program of claim 1, **characterized in that** after the preferred PEEP value for ventilation to the patient is determined according to the change in the at least one physiological parameter, the method further comprises:

providing mechanical ventilation to the patient according to the preferred PEEP value.

7. An apparatus (1) for determining positive end expiratory pressure applicable to a ventilation device, comprising:

a ventilation control module (10) for stepwise changing a PEEP value for ventilation of the ventilation device to a patient;
a physiological parameter monitoring module (11) for monitoring at least one physiological parameter of the patient; and
a calculation module (12) for calculating a change in the at least one physiological parameter every time the PEEP value changes and determining a preferred PEEP value for ventilation to the patient according to the change in the at least one physiological parameter;
**characterized in that**, the at least one physiological parameter comprises at least two of a driving pressure, a compliance, a dead space ratio and an oxygenation index, and the calculation module (12) is further configured for: determining respective candidate PEEP values according to the changes in the respective at least two physiological parameters; and determining the preferred PEEP value for ventilation to the patient according to the respective candidate PEEP values;
the candidate PEEP values comprise any one of the PEEP value corresponding to a minimum value of the driving pressure and the PEEP value corresponding to a maximum value of the compliance, and at least one of the PEEP value corresponding to a minimum value of the dead space ratio and the PEEP value corresponding to a maximum point of a change rate of the oxygenation index;
wherein the calculation module is configured for determining the preferred PEEP value for ventilation to the patient according to the respective candidate PEEP values by:
determining the preferred PEEP value according to the PEEP value corresponding to the minimum value of the dead space ratio and any one of the PEEP value corresponding to the minimum value of the driving pressure and the PEEP value corresponding to the maximum value of the compliance; or, determining the preferred PEEP value according to the PEEP value corresponding to the maximum point of the change rate of the oxygenation index and any one of the PEEP value corresponding to the minimum value of the driving pressure and the PEEP value corresponding to the maximum value of the compliance; or, determining the preferred PEEP value according to the PEEP value corresponding to the minimum value of the dead space ratio, the PEEP value corresponding to the maximum point of the change rate of the oxygenation index and any one of the PEEP value corresponding to the minimum value of the driving pressure and the PEEP value corresponding to the maximum value of the compliance.

8. The apparatus (1) of claim 7, **characterized in that** the at least one physiological parameter is the driving pressure, and
the calculation module (12) determines that the PEEP value corresponding to the minimum value of the driving pressure is the preferred PEEP value as the PEEP value changes stepwise.

9. The apparatus (1) of claim 7, **characterized in that** the at least one physiological parameter is the compliance, and the calculation module (12) determines that the PEEP value corresponding to the maximum value of the compliance is the preferred PEEP value as the PEEP value changes stepwise.

10. The apparatus (1) of claim 7, **characterized in that** the at least one physiological parameter is the dead space ratio, and
the calculation module (12) determines that the PEEP value corresponding to the minimum value of the dead space ratio is the preferred PEEP value as the PEEP value changes stepwise.

11. The apparatus (1) of claim 7, **characterized in that** the at least one physiological parameter is the oxygenation index, and
the calculation module (12) determines that the PEEP value corresponding to the maximum point of the change rate of the oxygenation index is the preferred PEEP value as the PEEP value changes stepwise.

12. The apparatus (1) of claim 7, **characterized by** further comprising: an output module (13), wherein the output module (13) outputs the preferred PEEP value according to a preset prompting strategy.

13. A ventilation device comprising an apparatus (1) for determining positive end expiratory pressure of any one of claims 7-12, **characterized by** comprising a gas source (3), an inspiratory branch (4), an expiratory branch (5), and a display (6), wherein

the gas source (3) provides a gas during mechanical ventilation;

the inspiratory branch (4) is connected to the gas source (3) and provides an inspiratory path during the mechanical ventilation;
the expiratory branch (5) provides an expiratory path during the mechanical ventilation;
the apparatus (1) for determining positive end expiratory pressure is connected to the inspiratory branch (4) and the expiratory branch (5);
the apparatus (1) for determining positive end expiratory pressure determines the preferred PEEP value during the mechanical ventilation; and
the display (6) is connected to the apparatus (1) for determining positive end expiratory pressure, and displays a respiratory waveform during the mechanical ventilation.

**Patentansprüche**

1. Computerprogramm zur Bestimmung des positiven Ausatmungsenddrucks mit Anweisungen, um ein Beatmungsgerät dazu zu veranlassen, die folgenden Schritte auszuführen:

   Steuerung eines Beatmungssteuermoduls (10) zur schrittweisen Änderung eines PEEP-Wertes für die Beatmung eines Patienten durch das Beatmungsgerät;
   Steuerung eines Moduls zur Überwachung physiologischer Parameter (11) zur Überwachung mindestens eines physiologischen Parameters des Patienten;
   Steuerung eines Berechnungsmoduls (12) zur Berechnung einer Änderung mindestens eines physiologischen Parameters bei jeder Änderung des PEEP-Wertes, und
   Bestimmung eines bevorzugten PEEP-Wertes für die Beatmung des Patienten entsprechend der Änderung mindestens eines physiologischen Parameters,
   **dadurch gekennzeichnet, dass** mindestens ein physiologischer Parameter zumindest zwei der folgenden Parameter umfasst:

   einen Antriebsdruck, eine Compliance, ein Totraumverhältnis und einen Oxygenierungsindex, und dass ein bevorzugter PEEP-Wert für die Beatmung des Patienten entsprechend der Änderung des zumindest einen physiologischen Parameters bestimmt wird, und zwar: durch Bestimmung der jeweiligen PEEP-Kandidatenwerte entsprechend den Änderungen der jeweiligen mindestens zwei physiologischen Parameter; und durch Bestimmung des bevorzugten PEEP-Wertes für die Beatmung des Patienten entsprechend den jeweiligen Kandidaten-PEEP-Werten;
   die Kandidaten-PEEP-Werte umfassen einen beliebigen PEEP-Wert, der einem Minimalwert des Antriebsdrucks entspricht, den PEEP-Wert, der einem Maximalwert der Compliance entspricht, sowie mindestens einen PEEP-Wert, der einem Minimalwert des Totraumverhältnisses entspricht, und den PEEP-Wert, der einem Maximalwert einer Änderungsrate des Oxygenierungsindexes entspricht,
   wobei die Bestimmung des bevorzugten PEEP-Wertes für die Beatmung des Patienten entsprechend den jeweiligen Kandidaten-PEEP-Werten Folgendes beinhaltet:

   die Bestimmung des bevorzugten PEEP-Werts entsprechend dem PEEP-Wert, der dem Minimalwert des Totraumverhältnisses entspricht, und einem beliebigen PEEP-Wert, der dem Minimalwert des Antriebsdrucks entspricht, oder dem PEEP-Wert, der dem Höchstwert der Compliance entspricht; oder, die Bestimmung des bevorzugten PEEP-Werts entsprechend dem PEEP-Wert, der dem Maximalwert der Änderungsrate des Oxygenierungsindex entspricht, und dem PEEP-Wert, der dem Minimalwert des Antriebsdrucks entspricht, sowie dem PEEP-Wert, der dem Maximalwert der Compliance entspricht; oder, die Bestimmung des bevorzugten PEEP-Wertes entsprechend dem PEEP-Wert, der dem Minimalwert des Totraumverhältnisses entspricht, dem PEEP-Wert, der dem Maximalpunkt der Änderungsrate des Oxygenierungsindexes entspricht, und dem PEEP-Wert, der dem Minimalwert des Antriebsdrucks entspricht, sowie dem PEEP-Wert, der dem Maximalwert der Compliance entspricht..

2. Computerprogramm nach Anspruch 1, **dadurch gekennzeichnet, dass** zumindest ein physiologischer Parameter dem Antriebsdruck entspricht; und
   das Bestimmen eines bevorzugten PEEP-Wertes für die Beatmung des Patienten entsprechend der Änderung zumindest eines physiologischen Parameters, Folgendes umfasst:
   Einstellen des PEEP-Werts, der dem Mindestwert des Antriebsdrucks entspricht, als bevorzugter PEEP-Wert, da sich der PEEP-Wert schrittweise ändert.

3. Computerprogramm nach Anspruch 1, **dadurch gekennzeichnet, dass** zumindest ein physiologischer Parameter dem Compliance entspricht, und

   das Bestimmen eines bevorzugten PEEP-Wertes für die Beatmung des Patients entsprechend der Änderung zumindest eines physiologischen Parameters, Folgendes umfasst:

   Einstellen des PEEP-Wertes, der dem Maximalwert der Compliance entspricht, als bevorzugter PEEP-Wert, da sich der PEEP-Wert schrittweise ändert

4. Computerprogramm nach Anspruch 1, **dadurch gekennzeichnet, dass** zumindest ein physiologischer Parameter dem Totraumverhältnis entspricht, und

   das Bestimmen eines bevorzugten PEEP-Wertes für die Beatmung des Patients entsprechend der Änderung zumindest eines physiologischen Parameters, Folgendes umfasst:

   Einstellen des PEEP-Werts, der dem Mindestwert des Totraumverhältnisses entspricht, als bevorzugter PEEP-Wert, da sich der PEEP-Wert schrittweise ändert

5. Computerprogramm nach Anspruch 1, **dadurch gekennzeichnet, dass** zumindest einer der physiologischen Parameter dem Oxygenierungsindex entspricht, und

   das Bestimmen eines bevorzugten PEEP-Wertes für die Beatmung des Patients entsprechend der Änderung zumindest eines physiologischen Parameters, Folgendes umfasst:

   Einstellen des PEEP-Werts, der dem Höchstwert der Änderungsrate des Oxygenierungsindex entspricht, als bevorzugten PEEP-Wert, da sich der PEEP-Wert schrittweise ändert..

6. Computerprogramm nach Anspruch 1, **dadurch gekennzeichnet, dass** das Verfahren, nachdem der bevorzugte PEEP-Wert für die Beatmung des Patienten gemäß der Änderung von mindestens einem physiologischen Parameter bestimmt wurde, ferner die mechanische Beatmung des Patienten entsprechend dem bevorzugten PEEP-Wert beinhaltet.

7. Vorrichtung (1) zur Bestimmung des positiven Ausatmungsenddrucks, der für ein Beatmungsgerät anwendbar ist, mit:

   ein Beatmungssteuermodul (10) zur schrittweisen Änderung eines PEEP-Wertes für die Beatmung des Patienten durch das Beatmungsgerät;

   ein Modul zur Überwachung physiologischer Parameter (11) zur Überwachung mindestens eines physiologischen Parameters des Patienten; und

   ein Berechnungsmodul (12) zur Berechnung einer Änderung von mindestens einem physiologischen Parameter jedes Mal, wenn sich der PEEP-Wert ändert, und zur Bestimmung eines bevorzugten PEEP-Wertes für die Beatmung des Patienten entsprechend der Änderung von mindestens einem der physiologischen Parameter; **dadurch gekennzeichnet, dass** mindestens ein physiologischer Parameter zumindest zwei der folgenden Parameter umfasst: einen Antriebsdruck, eine Compliance, ein Totraumverhältnis und einen Oxygenierungsindex, und das Berechnungsmodul (12) ferner dazu ausgelegt ist: die jeweiligen in Frage kommenden PEEP- Werte entsprechend den Änderungen der mindestens zwei jeweiligen physiologischen Parameter zu bestimmen; und den bevorzugten PEEP-Wert für die Beatmung des Patienten entsprechend den jeweiligen in Fragen kommenden PEEP-Werten zu bestimmen;

   die PEEP- Werte, die in Frage kommen, umfassen entweder den PEEP- Wert, der einem Minimalwert des Antriebsdrucks entspricht, den PEEP- Wert, der einem Maximalwert der Compliance entspricht, oder zumindest den PEEP- Wert, der einem Minimalwert des Totraumverhältnisses entspricht, und den PEEP- Wert, der einem Maximalwert einer Änderungsrate des Oxygenierungsindexes entspricht,

   wobei das Berechnungsmodul so konfiguriert ist, dass es den bevorzugten PEEP-Wert für die Beatmung des Patienten entsprechend den jeweiligen in Frage kommenden PEEP-Werten bestimmt, und zwar:

   das Bestimmen des bevorzugten PEEP-Werts anhand des PEEP-Werts, der dem Minimalwert des Totraumverhältnisses entspricht, und anhand des PEEP-Werts, der dem Minimalwert des Antriebsdrucks entspricht, sowie des PEEP-Werts, der dem Maximalwert der Compliance entsprich; oder, das Bestimmen des bevorzugten PEEP-Wertes anhand des PEEP-Wertes, der dem Maximalwert der Änderungsrate des Oxygenierungsindexes entspricht, und des PEEP-Wertes, der dem Minimalwert des Antriebsdrucks entspricht, sowie des PEEP-Wertes, der dem Maximalwert der Compliance entspricht; oder, das Bestimmen des bevorzugten PEEP-Wertes anhand des PEEP-Wertes, der dem Minimalwert des Totraumverhältnisses entspricht, des PEEP-Wertes, der dem Maximalpunkt der Änderungsrate des Oxygenierungsindexes entspricht, und des PEEP-Wertes, der dem Minimalwert des Antriebsdrucks entspricht, sowie des PEEP-Wertes, der dem Maximalwert der Compliance entspricht..

8. Vorrichtung (1) nach Anspruch 7, **dadurch gekennzeichnet, dass** mindestens ein physiologischer Parameter dem Antriebsdruck entspricht, und
das Berechnungsmodul (12) bestimmt, dass der PEEP-Wert, der dem Mindestwert des Antriebsdrucks entspricht, der bevorzugte PEEP-Wert ist, da sich der PEEP-Wert schrittweise ändert.

9. Vorrichtung (1) nach Anspruch 7, **dadurch gekennzeichnet, dass** mindestens ein physiologischer Parameter der Compliance entspricht, und
das Berechnungsmodul (12) bestimmt, dass der PEEP-Wert, der dem maximalen Wert der Compliance entspricht, der bevorzugte PEEP-Wert ist, da sich der PEEP-Wert schrittweise ändert..

10. Vorrichtung (1) nach Anspruch 7, **dadurch gekennzeichnet, dass** mindestens ein physiologischer Parameter dem Totraumverhältnis entspricht, und
das Berechnungsmodul (12) bestimmt, dass der PEEP-Wert, der dem Mindestwert des Totraumverhältnisses entspricht, der bevorzugte PEEP-Wert ist, da sich der PEEP-Wert schrittweise ändert.

11. Vorrichtung (1) nach Anspruch 7, **dadurch gekennzeichnet, dass** mindestens ein physiologischer Parameter dem Oxygenierungsindex entspricht, und
das Berechnungsmodul (12) bestimmt, dass der PEEP-Wert, der dem Maximalpunkt der Änderungsrate des Oxygenierungsindex entspricht, der bevorzugte PEEP-Wert ist, da sich der PEEP-Wert schrittweise ändert

12. Vorrichtung (1) nach Anspruch 7, **dadurch gekennzeichnet, dass** sie ferner Folgendes umfasst: ein Output-Modul (13), wobei das Ausgabemodul (13) den bevorzugten PEEP-Wert gemäß einer voreingestellten Prompting-Strategie ausgibt.

13. Beatmungsgerät mit einer Vorrichtung (1) zur Bestimmung des positiven Ausatmungsenddrucks nach einem der Ansprüche 7 bis 12, mit einer Gaszufuhr (3), einem Einatmungszweig (4), einem Ausatmungszweig (5) und einem Display (6), wobei

die Gaszufuhr (3) während der mechanischen Beatmung ein Gas liefert;
der Einatmungszweig (4) ist mit der Gaszufuhr (3) verbunden und dient als Einatmungsweg während der mechanischen Beatmung;
der Ausatmungszweig (5) dient als Ausatmungsweg während der mechanischen Beatmung;
die Vorrichtung (1) zur Bestimmung des positiven Ausatmungsenddrucks ist mit dem Einatmungs (4) - und dem Ausatmungszweig (5) verbunden,
das Gerät (1) zur Bestimmung des positiven Ausatmungsenddrucks ermittelt den bevorzugten PEEP-Wert während der mechanischen Beatmung; und
das Display (6) ist mit dem Gerät (1) verbunden, um den positiven Ausatmungsenddruck zu bestimmen, und zeigt eine Atmungskurve während der mechanischen Beatmung an.

**Revendications**

1. Programme informatique destiné à déterminer une pression positive en fin d'expiration comprenant des instructions pour amener un dispositif de ventilation à exécuter les étapes comprenant :

le fait de commander un module de commande de ventilation (10) destiné à modifier progressivement une valeur de pression positive en fin d'expiration (PEEP) pour une ventilation du dispositif de ventilation appliquée à un patient ;
le fait de commander un module de surveillance de paramètre physiologique (11) destiné à surveiller au moins un paramètre physiologique du patient ;
le fait de commander un module de calcul (12) destiné à calculer une variation dans le au moins un paramètre physiologique chaque fois que la valeur de PEEP est modifiée ; et
le fait de déterminer une valeur de PEEP préférée pour une ventilation appliquée au patient selon la variation dans le au moins un paramètre physiologique ;
**caractérisé en ce que**, le au moins un paramètre physiologique comprend au moins deux paramètres parmi une pression d'entraînement, une compliance, un rapport de volume mort et un indice d'oxygénation, et le fait de déterminer une valeur de PEEP préférée pour une ventilation appliquée au patient selon la variation dans le au moins un paramètre physiologique comprend : le fait de déterminer des valeurs de PEEP candidates respectives selon les variations dans les au moins deux paramètres physiologiques respectifs ; et le fait de déterminer la valeur de PEEP préférée pour une ventilation appliquée au patient selon les valeurs de PEEP candidates respectives ;

les valeurs de PEEP candidates comprennent une quelconque valeur parmi la valeur de PEEP correspondant à une valeur minimale de la pression d'entraînement et la valeur de PEEP correspondant à une valeur maximale de la compliance, et au moins une valeur parmi la valeur de PEEP correspondant à une valeur minimale du rapport de volume mort et la valeur de PEEP correspondant à un point maximal d'un taux de variation de l'indice d'oxygénation ;

où le fait de déterminer la valeur de PEEP préférée pour une ventilation appliquée au patient selon les valeurs de PEEP candidates respectives comprend :

le fait de déterminer la valeur de PEEP préférée selon la valeur de PEEP correspondant à la valeur minimale du rapport de volume mort et une quelconque valeur parmi la valeur de PEEP correspondant à la valeur minimale de la pression d'entraînement et la valeur de PEEP correspondant à la valeur maximale de la compliance ; ou, le fait de déterminer la valeur de PEEP préférée selon la valeur de PEEP correspondant au point maximal du taux de variation de l'indice d'oxygénation et une quelconque valeur parmi la valeur de PEEP correspondant la valeur minimale de la pression d'entraînement et la valeur de PEEP correspondant à la valeur maximale de la compliance ; ou, le fait de déterminer la valeur de PEEP préférée selon la valeur de PEEP correspondant à la valeur minimale du rapport de volume mort, la valeur de PEEP correspondant au point maximal du taux de variation de l'indice d'oxygénation et une quelconque valeur parmi la valeur de PEEP correspondant à la valeur minimale de la pression d'entraînement et la valeur de PEEP correspondant à la valeur maximale de la compliance.

2. Programme informatique selon la revendication 1, **caractérisé en ce que** le au moins un paramètre physiologique est la pression d'entraînement ; et

le fait de déterminer une valeur de PEEP préférée pour une ventilation appliquée au patient selon la variation dans le au moins un paramètre physiologique comprend :

le fait de fixer la valeur de PEEP correspondant à la valeur minimale de la pression d'entraînement comme étant la valeur de PEEP préférée lorsque la valeur de PEEP varie progressivement.

3. Programme informatique selon la revendication 1, **caractérisé en ce que** le au moins un paramètre physiologique est la compliance ; et

le fait de déterminer une valeur de PEEP préférée pour une ventilation appliquée au patient selon la variation dans le au moins un paramètre physiologique comprend :

le fait de fixer la valeur de PEEP correspondant à la valeur maximale de la compliance comme étant la valeur de PEEP préférée lorsque la valeur de PEEP varie progressivement.

4. Programme informatique selon la revendication 1, **caractérisé en ce que** le au moins un paramètre physiologique est le rapport de volume mort ; et

le fait de déterminer une valeur de PEEP préférée pour une ventilation appliquée au patient selon la variation dans le au moins un paramètre physiologique comprend :

le fait de fixer la valeur de PEEP correspondant à la valeur minimale du rapport de volume mort comme étant la valeur de PEEP préférée lorsque la valeur de PEEP varie progressivement.

5. Programme informatique selon la revendication 1, **caractérisé en ce que** le au moins un paramètre physiologique est l'indice d'oxygénation ; et

le fait de déterminer une valeur de PEEP préférée pour une ventilation appliquée au patient selon la variation dans le au moins un paramètre physiologique comprend :

le fait de fixer la valeur de PEEP correspondant au point maximal du taux de variation de l'indice d'oxygénation comme étant la valeur de PEEP préférée lorsque la valeur de PEEP varie progressivement.

6. Programme informatique selon la revendication 1, **caractérisé en ce que**, après que la valeur de PEEP préférée pour une ventilation appliquée au patient a été déterminée selon la variation dans le au moins un paramètre physiologique, le procédé comprend en outre :

le fait de fournir une ventilation mécanique au patient selon la valeur de PEEP préférée.

7. Appareil (1) destiné à déterminer une pression positive en fin d'expiration applicable à un dispositif de ventilation, comprenant :

un module de commande de ventilation (10) destiné à modifier progressivement une valeur de PEEP pour une ventilation du dispositif de ventilation appliquée à un patient ;

un module de surveillance de paramètre physiologique (11) destiné à surveiller au moins un paramètre physiologique du patient ; et

un module de calcul (12) destiné à calculer une variation dans le au moins un paramètre physiologique chaque fois que la valeur de PEEP est modifiée et à déterminer une valeur de PEEP préférée pour une ventilation appliquée au patient selon la variation dans le au moins un paramètre physiologique ;

**caractérisé en ce que**, le au moins un paramètre physiologique comprend au moins deux paramètres parmi une pression d'entraînement, une compliance, un rapport de volume mort et un indice d'oxygénation, et le module de calcul (12) est en outre configuré pour : déterminer des valeurs de PEEP candidates respectives selon les variations dans les au moins deux paramètres physiologiques respectifs ; et à déterminer la valeur de PEEP préférée pour une ventilation appliquée au patient selon les valeurs de PEEP candidates respectives ;

les valeurs de PEEP candidates comprennent une quelconque valeur parmi la valeur de PEEP correspondant à une valeur minimale de la pression d'entraînement et la valeur de PEEP correspondant à une valeur maximale de la compliance, et au moins une valeur parmi la valeur de PEEP correspondant à une valeur minimale du rapport de volume mort et la valeur de PEEP correspondant à un point maximal d'un taux de variation de l'indice d'oxygénation ;

où le module de calcul est configuré pour déterminer la valeur de PEEP préférée pour une ventilation appliquée au patient selon les valeurs de PEEP candidates respectives en :

déterminant la valeur de PEEP préférée selon la valeur de PEEP correspondant à la valeur minimale du rapport de volume mort et une quelconque valeur parmi la valeur de PEEP correspondant à la valeur minimale de la pression d'entraînement et la valeur de PEEP correspondant à la valeur maximale de la compliance ; ou, en déterminant la valeur de PEEP préférée selon la valeur de PEEP correspondant au point maximal du taux de variation de l'indice d'oxygénation et une quelconque valeur parmi la valeur de PEEP correspondant la valeur minimale de la pression d'entraînement et la valeur de PEEP correspondant à la valeur maximale de la compliance ; ou, en déterminant la valeur de PEEP préférée selon la valeur de PEEP correspondant à la valeur minimale du rapport de volume mort, la valeur de PEEP correspondant au point maximal du taux de variation de l'indice d'oxygénation et une quelconque valeur parmi la valeur de PEEP correspondant à la valeur minimale de la pression d'entraînement et la valeur de PEEP correspondant à la valeur maximale de la compliance.

8. Appareil (1) selon la revendication 7, **caractérisé en ce que** le au moins un paramètre physiologique est la pression d'entraînement, et

le module de calcul (12) détermine que la valeur de PEEP correspondant à la valeur minimale de la pression d'entraînement est la valeur de PEEP préférée lorsque la valeur de PEEP varie progressivement.

9. Appareil (1) selon la revendication 7, **caractérisé en ce que** le au moins un paramètre physiologique est la compliance, et

le module de calcul (12) détermine que la valeur de PEEP correspondant à la valeur maximale de la compliance est la valeur de PEEP préférée lorsque la valeur de PEEP varie progressivement.

10. Appareil (1) selon la revendication 7, **caractérisé en ce que** le au moins un paramètre physiologique est le rapport de volume mort, et

le module de calcul (12) détermine que la valeur de PEEP correspondant à la valeur minimale du rapport de volume mort est la valeur de PEEP préférée lorsque la valeur de PEEP varie progressivement.

11. Appareil (1) selon la revendication 7, **caractérisé en ce que** le au moins un paramètre physiologique est l'indice d'oxygénation, et

le module de calcul (12) détermine que la valeur de PEEP correspondant au point maximal du taux de variation de l'indice d'oxygénation est la valeur de PEEP préférée lorsque la valeur de PEEP varie progressivement.

12. Appareil (1) selon la revendication 7, **caractérisé par** le fait de comprendre en outre : un module de sortie (13), où le module de sortie (13) délivre en sortie la valeur de PEEP préférée selon une stratégie de sollicitation préétablie.

13. Dispositif de ventilation comprenant un appareil (1) destiné à déterminer une pression positive en fin d'expiration selon l'une quelconque des revendications 7 à 12, **caractérisé par** le fait de comprendre une source de gaz (3), une branche inspiratoire (4), une branche expiratoire (5), et un affichage (6), où

la source de gaz (3) fournit un gaz durant une ventilation mécanique ;

la branche inspiratoire (4) est reliée à la source de gaz (3) et fournit un trajet inspiratoire durant la ventilation mécanique ;

la branche expiratoire (5) fournit un trajet expiratoire durant la ventilation mécanique ;

l'appareil (1) destiné à déterminer une pression positive en fin d'expiration est relié à la branche inspiratoire (4) et à la branche expiratoire (5) ;

l'appareil (1) destiné à déterminer une pression positive en fin d'expiration détermine la valeur de PEEP préférée durant la ventilation mécanique ; et

l'affichage (6) est relié à l'appareil (1) destiné à déterminer une pression positive en fin d'expiration, et affiche une forme d'onde respiratoire durant la ventilation mécanique.

Stepwise change a PEEP value for ventilation of a ventilation device to a patient ⟶ S101

Monitor at least one physiological parameter of the patient ⟶ S102

Calculate a change in the at least one physiological parameter every time the PEEP value changes ⟶ S103

Determine a preferred PEEP value for ventilation to the patient according to the change in the at least one physiological parameter ⟶ S104

*FIG. 1*

First coordinate system

30 cm H₂O

15 cm H₂O

0

PEEP=0
$P_{ploot}$=0

PEEP=2
$P_{plat}$=15
ΔPEEP> ΔPploot

PEEP=4
$P_{plat}$=16
ΔPEEP> ΔPplaot

PEEP=6
$P_{plat}$=18
ΔPEEP= ΔPplaat

PEEP=8
$P_{plat}$=21
ΔPEEP< ΔPplaot

15      16      18      21

First respiratory cycle      Second respiratory cycle      Third respiratory cycle      Fourth respiratory cycle      Fifth respiratory cycle      t

Second coordinate system

0                                                                          t

*FIG. 2*

*FIG. 3*

*FIG. 4*

A ventilation control module of an apparatus for determining positive end expiratory pressure stepwise changes a PEEP value for ventilation of a ventilation device to a patient ⎯ S201

A physiological parameter detection module of the apparatus for determining positive end expiratory pressure monitors a driving pressure of the patient ⎯ S202

A calculation module of the apparatus for determining positive end expiratory pressure calculates a change in the driving pressure every time the PEEP value changes ⎯ S203

The calculation module of the apparatus for determining positive end expiratory pressure determines that the PEEP value corresponding to a minimum value of the driving pressure is the preferred PEEP value as the PEEP value changes stepwise ⎯ S204

An output module of the apparatus for determining positive end expiratory pressure outputs the preferred PEEP value according to a preset prompting strategy ⎯ S205

The ventilation control module of the apparatus for determining positive end expiratory pressure provides mechanical ventilation to the patient according to the preferred PEEP value ⎯ S206

*FIG. 5*

A ventilation control module of an apparatus for determining positive end expiratory pressure stepwise changes a PEEP value for ventilation of a ventilation device to a patient ⎯ S301

A physiological parameter detection module of the apparatus for determining positive end expiratory pressure monitors a compliance of the patient ⎯ S302

A calculation module of the apparatus for determining positive end expiratory pressure calculates a change in the compliance every time the PEEP value changes ⎯ S303

A calculation module of the apparatus for determining positive end expiratory pressure determines that the PEEP value corresponding to a maximum compliance value is the preferred PEEP value as the PEEP value changes stepwise ⎯ S304

An output module of the apparatus for determining positive end expiratory pressure outputs the preferred PEEP value according to a preset prompting strategy ⎯ S305

The ventilation control module of the apparatus for determining positive end expiratory pressure provides mechanical ventilation to the patient according to the preferred PEEP value ⎯ S306

*FIG. 6*

A ventilation control module of an apparatus for determining positive end expiratory pressure stepwise changes a PEEP value for ventilation of a ventilation device to a patient — S401

A physiological parameter detection module of the apparatus for determining positive end expiratory pressure monitors a dead space ratio of the patient — S402

A calculation module of the apparatus for determining positive end expiratory pressure calculates a change in the dead space ratio every time the PEEP value changes — S403

The calculation module of the apparatus for determining positive end expiratory pressure determines that the PEEP value corresponding to a minimum value of the dead space ratio is the preferred PEEP value as the PEEP value changes stepwise — S404

An output module of the apparatus for determining positive end expiratory pressure outputs the preferred PEEP value according to a preset prompting strategy — S405

The ventilation control module of the apparatus for determining positive end expiratory pressure provides mechanical ventilation to the patient according to the preferred PEEP value — S406

**FIG. 7**

A ventilation control module of an apparatus for determining positive end expiratory pressure stepwise changes a PEEP value for ventilation of a ventilation device to a patient — S501

A physiological parameter detection module of the apparatus for determining positive end expiratory pressure monitors an oxygenation index of the patient — S502

A calculation module of the apparatus for determining positive end expiratory pressure calculates a change in the oxygenation index every time the PEEP value changes — S503

The calculation module of the apparatus for determining positive end expiratory pressure determines that the PEEP value corresponding to a maximum point of the rate of change in the oxygenation index is the preferred PEEP value as the PEEP value changes stepwise — S504

An output module of the apparatus for determining positive end expiratory pressure outputs the preferred PEEP value according to a preset prompting strategy — S505

The ventilation control module of the apparatus for determining positive end expiratory pressure provides mechanical ventilation to the patient according to the preferred PEEP value — S506

**FIG. 8**

Apparatus for determining positive end expiratory pressure 1

Ventilation control module 10

Physiological parameter monitoring module 11

Calculation module 12

FIG. 9

Apparatus for determining positive end expiratory pressure 1

Ventilation control module 10

Physiological parameter monitoring module 11

Calculation module 12

Output module 13

FIG. 10

FIG. 11

FIG. 12

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2246087 A1
- WO 2018153964 A
- EP 1579882 A1
- US 20120055476 A
- US 20050109340 A